# EUROPEAN PATENT APPLICATION

(11) **EP 2 998 298 A1**
(43) Date of publication of application: **23.03.2016**
(21) Application number: 14819916.9
(22) Date of filing: 26.06.2014
(51) Int. Cl.: C07D 233/61, A61K 31/4178, A61K 31/454, A61K 31/496, A61K 31/5377, A61P 27/02, A61P 29/00, A61P 39/06, C07D 403/10, C07D 471/08

(54) **PHENYLIMIDAZOLE DERIVATIVE, AND THERAPEUTIC MEDICINE OR PREVENTIVE MEDICINE FOR INFLAMMATORY DISEASE, ETC.**

(30) Priority: 04.07.2013 JP 2013140342
(71) Applicant: Nippon Soda Co., Ltd., Tokyo 100-8165 (JP)
(72) Inventor: UMEDA, Nobuhiro, Odawara-shi Kanagawa 250-0280 (JP); TSUBOKURA, Shiro, Takaoka-shi Toyama 933-8507 (JP); UCHIDA, Seiichi, Odawara-shi Kanagawa 250-0280 (JP); KOIZUMI, Keiji, Odawara-shi Kanagawa 250-0280 (JP); MOROE, Hiroko, Odawara-shi Kanagawa 250-0280 (JP); OHSHIO, Ichiro, Odawara-shi Kanagawa 250-0280 (JP)
(74) Representative: Cabinet Plasseraud
(86) International application number: PCT/JP2014/067003
(87) International publication number: WO 2015/002061

(57) **Abstract**

A medicinal active ingredient is provided which is useful for the treatment or prevention of inflammatory diseases, diseases caused by lipid oxidation, retinochoroidal disorders. Aphenylimidazole derivative represented by Formula (IB) or a salt thereof is provided. A therapeutic medicine or preventive medicine is provided for inflammatory diseases, diseases caused by lipid oxidation, or retinochoroidal disorders, the medicine including at least one selected from the phenylimidazole derivative, a salt thereof, and metabolites thereof as an active ingredient. In Formula (IB), R^{1a} represents an alkyl group etc., R² represents an amino group etc., R³ represents a halogen atom, an alkyl group etc., R⁴ represents a cyano group etc., R⁵ represents an alkyl group etc., and R⁶ represents an alkyl group etc.

## Description

### Technical Field

The present invention relates to a phenylimidazole derivative and therapeutic medicine or preventive medicine for inflammatory diseases, diseases caused by lipid oxidation, or retinochoroidal disorders, including at least one selected from the derivative as an active ingredient.

Priority is claimed on Japanese Patent Application No. 2013-140342, filed on July 4, 2013, the content of which is incorporated herein by reference.

### Background Art

Inflammation is tissue reaction and systemic reaction against invasion to the body. Examples of invasion that cause inflammation include external stimuli such as wounds, infections, intrusion of antigenic substances (allergic reactions), and internal stimuli generated in the body such as cell injury. In addition, inflammation advances and worsens due to prostaglandin or leukotriene, both mediators of inflammation. An inflammation reaction is an important physiological reaction, but an extreme inflammation reaction causes significant damage to the body. For this reason, various kinds of anti-inflammatory agents have been developed. Anti-inflammatory agents currently in use are generally classified as either non-steroidal anti-inflammatory agents or steroidal anti-inflammatory agents. A non-steroidal anti-inflammatory agent is operated by biosynthesis inhibition of prostaglandin due to inhibition of activity of cyclooxygenase. Meanwhile, a steroidal anti-inflammatory agent binds to a receptor in cytoplasm and incorporated into the nucleus, activates specific genes, and induces biosynthesis of lipocortin. It is known that lipocortin shows anti-inflammatory actions through inhibition of various chemical mediators. The actions include cyclooxygenase pathway inhibition through phospholipase A2 inhibition, or a lipoxygenase pathway inhibition through leukotriene B4 inhibition.

Meanwhile, when a retina receives light from the outside, the energy thereof is absorbed by visual substances (rhodopsin) which are present in inner segments and outer segments of visual cells and converted to an electrical signal. A conduction path of vision in cells is visual cells, bipolar cells, and ganglion cells in order. After the electrical signal passes through a path of vision, the electrical signal is transmitted to a visual cortex in the occipital lobe of the cerebrum, and is then perceived as an image. In outer segments of visual cells in which visual substances are present, since a large amount of higher unsaturated fatty acids such as docosahexaenoic acid and arachidonic acid are present, the visual cells tend to be easily degenerated. Further, since degeneration of visual cells is almost irreversible, the degeneration is a factor of serious vision impairment. The degeneration of vision cells occurs for genetic reasons, but it is also known that visual cells degenerate due to various kinds of oxidation stresses such as light (ultraviolet rays), iron, oxygen, and radiation. Particularly, light received by the eyes throughout life is a typical example of an oxidation stress in retinas and is considered to be a main factor of retinochoroidal disorders.

A retinochoroidal disorder, especially a typical retinochoroidal disease accompanied with retinal light disorders, includes age-related macular degeneration (hereinafter, referred to as "AMD"). AMD is a disease that causes degeneration of a macular region of a retina with age and is continuously increasing with the advancement of an aging society. Particularly, in the west, AMD is highly ranked as a cause of late-eye blindness. AMD is divided into exudative AMD (hereinafter, referred to as "Wet AMD") which causes subretinal hemorrhage, edema, and serous retinal detachment accompanied by newborn blood vessels generated from choroid and atrophy AMD (hereinafter, referred to as "Dry AMD") that shows map-like atrophy lesions of retinal pigment epithelial cells or choriocapillaris which are not accompanied by newborn blood vessels generated from choroid. In addition, Wet AMD progresses rapidly and the prognosis worsens. Further, Wet AMD causes serious visual impairment. Meanwhile, Dry AMD progresses slowly, but causes advanced visual impairment when the lesions spread into macular regions. Moreover, it is considered that both are progressive types and highly likely to be binocular.

For treatments of Wet AMD, photodynamic therapy, laser treatments (laser photocoagulation), and surgical treatments (neovascular removal therapy and central fovea moving therapy) are used, but there are various problems in the prognosis and treatment satisfaction is extremely low. Moreover, as medical treatments, therapeutic medicine related to VEGF has also been developed, but the therapeutic effects are not satisfactory because of the administration route and side effects. Further, in regard to Dry AMD, medical treatments are almost not performed.

In addition, as medicine used for treatments of retinochoroidal disorders, PTL 1 or 2 discloses a dihydrobenzofuran derivative having an excellent antioxidant action and excellent tissue migration properties.

### Citation List

### Patent Literature

[PTL 1] WO2007/052794A
[PTL 2] WO2009/133701A

### Summary of Invention

### Technical Problem

Non-steroidal anti-inflammatory agents are generally used because of high safety, but the anti-inflammatory action is not necessarily strong. Meanwhile, steroidal anti-inflammatory agents show a significant inhibitory action with respect to inflammation and are also effective in autoimmune diseases, but the side effects thereof are problematic. For this reason, development of an anti-inflammatory agent whose action is strong and which has high safety has been required. An object of the present invention is to provide an active ingredient of a pharmaceutical product which is useful for treatment or prevention of diseases caused by an inflammatory reaction.

As cell membrane disorders, particularly, nerve cell membrane disorders, disorders caused by cerebral ischemia, excitotoxicity, or Aβ-toxicity are exemplary examples. Further, in regard to the cerebral ischemia and excitotoxicity, disorders accompanied by stroke, cerebral infarction, or cerebral embolism are exemplary examples. Moreover, 12/15-lipoxygenase and lipid peroxidase are enzymes that promote oxidation of lipids, and it is reported that amyloid beta which is a causative substance of Alzheimer's disease is excessively generated due to acceleration of 12/15-lipoxygenase (NIH Public Access, 2012; vol. 71; pp. 56 to 67). Moreover, it is considered that 12/15-lipoxygenase is involved in the progress of arteriosclerosis by oxidizing esterified polyunsaturated fatty acids of low-density lipoprotein (LDL) (The Japanese Pharmacological Society 2004; 124; 415 to 425). An object of the present invention is to provide an active ingredient of a pharmaceutical product which is useful for treatment or prevention of diseases caused by lipid oxidation.

In the derivative described in PTL 1 or 2, there is a variation in treatment effects or preventive effects in experiments performed on animal models of retinal light disorders. Another object of the present invention is to provide an active ingredient of a pharmaceutical product which is useful for treatment or prevention of retinochoroidal disorders.

### Solution to Problem

As a result of examination performed by the present inventors in order to solve the above-described problems, they found a phenylimidazole derivative having a specific structure. It is found that effects in a treatment or prevention of inflammatory diseases, diseases caused by lipid oxidation, or retinochoroidal disorders are significant when the derivative or a salt thereof is contained in medicines as an active ingredient. The present invention has been completed based on this knowledge.

The present invention includes the following aspects.
[1] A phenylimidazole derivative represented by Formula (I) or (II), or a salt thereof.

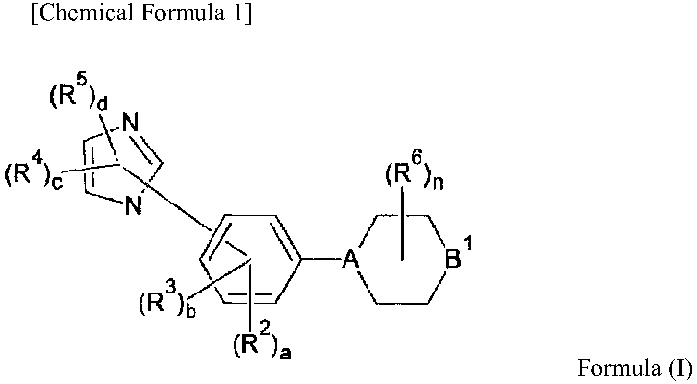

(In Formula (I), A represents a carbon atom or a nitrogen atom; B¹ represents a carbonyl group, a group represented by N-COR^{1a}, a group represented by N-C(R^{1a})=NOH, or a group represented by N-SO₂R^{1b}; R^{1a} represents a hydrogen atom, an alkyl group having 1 to 6 carbon atoms, an alkyl group which is substituted with G¹ and has 1 to 6 carbon atoms, an alkenyl group having 2 to 6 carbon atoms, an alkenyl group which is substituted with G1 and has 2 to 6 carbon atoms, an alkynyl group having 2 to 6 carbon atoms, an alkynyl group which is substituted with G¹ and has 2 to 6 carbon atoms, a cycloalkyl group having 3 to 8 carbon atoms, a cycloalkyl group which is substituted with G¹ and has 3 to 8 carbon atoms, a hydroxyl group, an alkoxy group having 1 to 6 carbon atoms, an alkoxy group which is substituted with G¹ and has 1 to 6 carbon atoms, an alkenyloxy group having 2 to 6 carbon atoms, an alkenyloxy group which is substituted with G¹ and has 2 to 6 carbon atoms, an alkynyloxy group having 2 to 6 carbon atoms, an alkynyloxy group which is substituted with G¹ and has 2 to 6 carbon atoms, an amino group, an amino group substituted with one G², or an amino group substituted with two G²s which are the same as or different from each other (in a case where the amino group is substituted with two G²s, two G²s may be bonded to each other to form a ring); R^{1b} represents an alkyl group having 1 to 6 carbon atoms, an alkyl group which is substituted with G¹ and has 1 to 6 carbon atoms, an alkenyl group having 2 to 6 carbon atoms, an alkenyl group which is substituted with G² and has 2 to 6 carbon atoms, an alkynyl group having 2 to 6 carbon atoms, an alkynyl group which is substituted with G¹ and has 2 to 6 carbon atoms, a cycloalkyl group having 3 to 8 carbon atoms, a cycloalkyl group which is substituted with G¹ and has 3 to 8 carbon atoms, a hydroxyl group, an alkoxy group having 1 to 6 carbon atoms, an alkoxy group which is substituted with G¹ and has 1 to 6 carbon atoms, an alkenyloxy group having 2 to 6 carbon atoms, an alkenyloxy group which is substituted with G¹ and has 2 to 6 carbon atoms, an alkynyloxy group having 2 to 6 carbon atoms, an alkynyloxy group which is substituted with G¹ and has 2 to 6 carbon atoms, an amino group, an amino group substituted with one G², or an amino group substituted with two G²s which are the same as or different from each other (in a case where the amino group is substituted with two G²s, two G²s may be bonded to each other to form a ring); G¹ represents a halogen atom, a hydroxyl group, an alkoxy group having 1 to 6 carbon atoms, an amino group, or an amino group substituted with an alkyl group having 1 to 6 carbon atoms; G² represents an alkyl group having 1 to 6 carbon atoms, an alkyl group which is substituted with G¹ and has 1 to 6 carbon atoms, an alkenyl group having 2 to 6 carbon atoms, an alkenyl group which is substituted with G¹ and has 2 to 6 carbon atoms, an alkynyl group having 2 to 6 carbon atoms, an alkynyl group which is substituted with G¹ and has 2 to 6 carbon atoms, a cycloalkyl group having 3 to 8 carbon atoms, a cycloalkyl group which is substituted with G¹ and has 3 to 8 carbon atoms, an alkylidene group having 1 to 6 carbon atoms, an alkylidene group which is substituted with G¹ and has 1 to 6 carbon atoms, a formyl group, an alkylcarbonyl group having 1 to 6 carbon atoms, an alkylcarbonyl group which is substituted with G¹ and has 1 to 6 carbon atoms, a cycloalkylcarbonyl group having 3 to 8 carbon atoms, a cycloalkylcarbonyl group which is substituted with G¹ and has 3 to 8 carbon atoms, an alkoxycarbonyl group having 1 to 6 carbon atoms, an alkoxycarbonyl group which is substituted with G¹ and has 1 to 6 carbon atoms, an alkylsulfonyl group having 1 to 6 carbon atoms, or an alkylsulfonyl group which is substituted with G¹ and has 1 to 6 carbon atoms; R² represents an amino group, an amino group substituted with one G², an amino group substituted with two G²s which are the same as or different from each other, a hydroxyl group, an alkoxy group having 1 to 6 carbon atoms, an alkylcarbonyloxy group having 1 to 6 carbon atoms, an alkoxycarbonyloxy group having 1 to 6 carbon atoms, a cyano group, or an alkyl group which is substituted with G³ and has 1 to 6 carbon atoms; G³ represents an amino group, an amino group substituted with one G², an amino group substituted with two G²s which are the same as or different from each other, a hydroxyl group, an alkoxy group having 1 to 6 carbon atoms, an alkylcarbonyloxy group having 1 to 6 carbon atoms, an alkoxycarbonyloxy group having 1 to 6 carbon atoms, or a cyano group; a represents an integer of 1 to 4, and when a represents 2 or greater, R²s may be the same as or different from each other; R³ represents a halogen atom or an organic group other than G³; b represents an integer of 0 to 3, and when b represents 2 or greater, R³s may be the same as or different from each other, provided that a relationship of "a + b ≤ 4" is satisfied; R⁴ represents a cyano group or an alkyl group which is substituted with G³ and has 1 to 6 carbon atoms; c represents an integer of 0 to 3, and when c represents 2 or greater, R⁴s may be the same as or different from each other; R⁵ represents a halogen atom or an organic group other than G³; d represents an integer of 0 to 3, and when d represents 2 or greater, R⁵s may be the same as or different from each other, provided that a relationship of "c + d ≤ 3" is satisfied; R⁶ represents an alkyl group having 1 to 6 carbon atoms; and n represents an integer of 0 to 4, and when n represents 2 or greater, R⁶s may be the same as or different from each other and two R⁶s may be bonded to each other to form an alkylene group having 2 to 6 carbon atoms.)

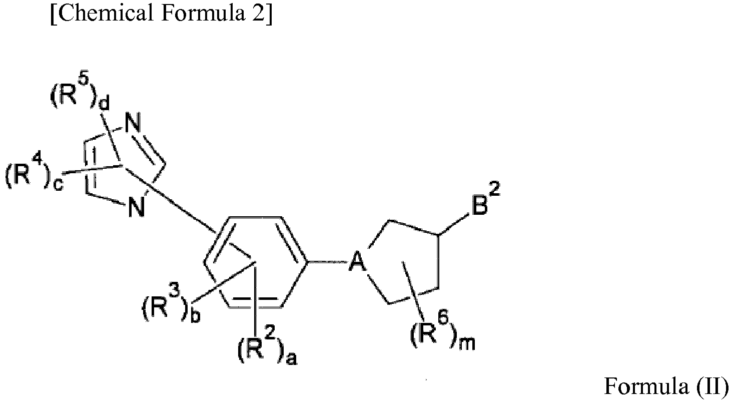

(In Formula (II), B² represents a group represented by NR^{1c}-COR^{1a}, a group represented by NR^{1c}-C(R^{1a})=NOH, or a group represented by NR^{1c}-SO₂R^{1b}; R^{1c} represents a hydrogen atom or an alkyl group having 1 to 6 carbon atoms; A, R^{1a}, R^{1b}, R², a, R³, b, R⁴, c, R⁵, d, and R⁶ have the same definitions as those in Formula (I); m represents an integer of 0 to 3, and when m represents 2 or greater, R⁶s may be the same as or different from each other and two R⁶s may be bonded to each other to form an alkylene group having 2 to 6 carbon atoms.)

[2] The phenylimidazole derivative according to [1] or a salt thereof, wherein, in Formula (I), A represents a nitrogen atom; B¹ represents a group represented by N-COR^{1a}; and the imidazolyl group which is a substituent of the benzene ring is an imidazole-1-yl group.
[3] The phenylimidazole derivative according to [1] or a salt thereof, wherein, in Formula (I), A represents a nitrogen atom; B¹ represents a group represented by N-COR^{1a}; the imidazolyl group which is a substituent of the benzene ring is an imidazole-1-yl group; and the imidazolyl group is in a meta-position with respect to a piperazine ring.
[4] The phenylimidazole derivative according to [1] or a salt thereof, wherein, in Formula (II), A represents a nitrogen atom; B² represents a group represented by NR^{1c}-COR^{1a}; and the imidazolyl group which is a substituent of the benzene ring is an imidazole-1-yl group.
[5] The phenylimidazole derivative according to [1] or a salt thereof, wherein, in Formula (II), A represents a nitrogen atom; B² represents a group represented by NR^{1c}-COR^{1a}; the imidazolyl group which is a substituent of the benzene ring is an imidazole-1-yl group; and the imidazolyl group is in a meta-position with respect to a pyrrolidine ring.
[6] A therapeutic medicine or preventive medicine for inflammatory diseases, diseases caused by lipid oxidation, or retinochoroidal disorders, the medicine comprising at least one selected from the phenylimidazole derivative according to anyone of [1] to [5], a salt thereof, and metabolites thereof, as an active ingredient.
[7] The therapeutic medicine or preventive medicine according to [6], further comprising a pharmacologically acceptable additive.
[8] The therapeutic medicine or preventive medicine according to [6], wherein the retinochoroidal disorder is age-related macular degeneration, diabetic retinopathy, or diabetic macular edema.
[9] Use of at least one selected from the phenylimidazole derivative according to any one of [1] to [5] and a salt thereof for treating or preventing inflammatory diseases, diseases caused by lipid oxidation, or retinochoroidal disorders. Advantageous Effects of Invention

Since a phenylimidazole derivative of the present invention and a salt thereof inhibits respective enzyme activities of leukotriene A4 hydrolase, leukotriene C4 synthase, thromboxane synthase, phospholipase A2-II, and MAP kinase I, the phenylimidazole derivative or a salt thereof is useful as an active ingredient of therapeutic medicine or preventive medicine for various inflammatory diseases. In addition, since the phenylimidazole derivative or a salt thereof inhibits activities of 15-lipoxygenase and lipid peroxidase, the phenylimidazole derivative or a salt thereof is useful as an active ingredient of therapeutic medicine or preventive medicine for various diseases caused by lipid oxidation.

Further, since the phenylimidazole derivative of the present invention or a salt thereof exhibits effects of a treatment or prevention of retinochoroidal disorders, particularly, retinal light disorders, the phenylimidazole derivative or a salt thereof is useful as an active ingredient of therapeutic medicine or preventive medicine of retinal light disorders.

The therapeutic medicine or preventive medicine according to the present invention is expected to exhibit therapeutic effect or preventive effect for retinal light disorders, with maintaining excellent tissue migration properties by oral administration.

### Brief Description of Drawings

FIG. 1 is a view showing a horizontal meridian section of an eye.
FIG. 2 is a view showing a posterior pole of an eye.
FIG. 3 is a view showing each layer of optic part of retina.

### Description of Embodiments

Aphenylimidazole derivative is a compound represented by Formula (I) or (II). Further, a salt of the phenylimidazole derivative is a salt of the compound represented by Formula (I) or (II).

### [A, B¹, B²]

A in Formula (I) or (II) represents a carbon atom or a nitrogen atom.
B¹ in Formula (I) represents a carbonyl group, a group represented by N-COR^{1a}, a group represented by N-C(R^{1a})=NOH, or a group represented by N-SO₂R^{1b}. Among these, a group represented by N-COR^{1a} is preferable.
B² in Formula (II) represents a group represented by NR^{1c}-COR^{1a}, a group represented by NR^{1c}-C(R^{1a})=NOH, or a group represented by NR^{1c}-SO₂R^{1b}. Among these, a group represented by NR^{1c}-COR^{1a} is preferable.

R^{1a} in B¹ or B² represents a hydrogen atom, an alkyl group having 1 to 6 carbon atoms, an alkyl group which is substituted with G¹ and has 1 to 6 carbon atoms, an alkenyl group having 2 to 6 carbon atoms, an alkenyl group which is substituted with G¹ and has 2 to 6 carbon atoms, an alkynyl group having 2 to 6 carbon atoms, an alkynyl group which is substituted with G¹ and has 2 to 6 carbon atoms, a cycloalkyl group having 3 to 8 carbon atoms, a cycloalkyl group which is substituted with G¹ and has 3 to 8 carbon atoms, a hydroxyl group, an alkoxy group having 1 to 6 carbon atoms, an alkoxy group which is substituted with G¹ and has 1 to 6 carbon atoms, an alkenyloxy group having 2 to 6 carbon atoms, an alkenyloxy group which is substituted with G¹ and has 2 to 6 carbon atoms, an alkynyloxy group having 2 to 6 carbon atoms, an alkynyloxy group which is substituted with G¹ and has 2 to 6 carbon atoms, an amino group, an amino group substituted with one G², or an amino group substituted with two G²s which are the same as or different from each other. Here, in this case where the amino group is substituted with two G²s, two G²s may be bonded to each other to form a ring.

R^{1b} in B¹ or B² represents an alkyl group having 1 to 6 carbon atoms, an alkyl group which is substituted with G¹ and has 1 to 6 carbon atoms, an alkenyl group having 2 to 6 carbon atoms, an alkenyl group which is substituted with G¹ and has 2 to 6 carbon atoms, an alkynyl group having 2 to 6 carbon atoms, an alkynyl group which is substituted with G¹ and has 2 to 6 carbon atoms, a cycloalkyl group having 3 to 8 carbon atoms, a cycloalkyl group which is substituted with G¹ and has 3 to 8 carbon atoms, a hydroxyl group, an alkoxy group having 1 to 6 carbon atoms, an alkoxy group which is substituted with G¹ and has 1 to 6 carbon atoms, an alkenyloxy group having 2 to 6 carbon atoms, an alkenyloxy group which is substituted with G¹ and has 2 to 6 carbon atoms, an alkynyloxy group having 2 to 6 carbon atoms, an alkynyloxy group which is substituted with G¹ and has 2 to 6 carbon atoms, an amino group, an amino group substituted with one G², or an amino group substituted with two G²s which are the same as or different from each other. Here, in a case where the amino group is substituted with two G²s, two G²s may be bonded to each other to form a ring.

R^{1C} in B² represents a hydrogen atom or an alkyl group having 1 to 6 carbon atoms.

G¹ in R^{1a} and R^{1b} represents a halogen atom, a hydroxyl group, an alkoxy group having 1 to 6 carbon atoms, an amino group, or an amino group substituted with an alkyl group having 1 to 6 carbon atoms.

Examples of the "halogen atom" in G¹ include a fluorine atom, a chlorine atom, a bromine atom, and an iodine atom.

Examples of the "alkoxy group having 1 to 6 carbon atoms" in G¹ include a methoxy group, an ethoxy group, a n-propoxy group, a n-butoxy group, a n-pentyloxy group, a n-hexyloxy group, an i-propoxy group, an i-butoxy group, a s-butoxy group, a t-butoxy group, and an i-hexyloxy group.

Examples of the "amino group substituted with an alkyl group having 1 to 6 carbon atoms" in G¹ include a methylamino group, a dimethylamino group, and a diethylamino group.

G² in R^{1a} and R^{1b} represents an alkyl group having 1 to 6 carbon atoms, an alkyl group which is substituted with G¹ and has 1 to 6 carbon atoms, an alkenyl group having 2 to 6 carbon atoms, an alkenyl group which is substituted with G¹ and has 2 to 6 carbon atoms, an alkynyl group having 2 to 6 carbon atoms, an alkynyl group which is substituted with G¹ and has 2 to 6 carbon atoms, a cycloalkyl group having 3 to 8 carbon atoms, a cycloalkyl group which is substituted with G¹ and has 3 to 8 carbon atoms, an alkylidene group having 1 to 6 carbon atoms, an alkylidene group which is substituted with G¹ and has 1 to 6 carbon atoms, a formyl group, an alkylcarbonyl group having 1 to 6 carbon atoms, an alkylcarbonyl group which is substituted with G¹ and has 1 to 6 carbon atoms, a cycloalkylcarbonyl group having 3 to 8 carbon atoms, a cycloalkylcarbonyl group which is substituted with G¹ and has 3 to 8 carbon atoms, an alkoxycarbonyl group having 1 to 6 carbon atoms, an alkoxycarbonyl group which is substituted with G¹ and has 1 to 6 carbon atoms, an alkylsulfonyl group having 1 to 6 carbon atoms, or an alkylsulfonyl group which is substituted with G¹ and has 1 to 6 carbon atoms. G¹ is the same as defined above and examples thereof include the same as those described above for G¹.

Examples of the "alkyl group having 1 to 6 carbon atoms" in G² include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, a 2,2-dimethylpropyl group, and an i-hexyl group.

Examples of the "alkyl group which is substituted with G¹ and has 1 to 6 carbon atoms" in G² include a haloalkyl group having 1 to 6 carbon atoms such as a fluoromethyl group, a chloromethyl group, a bromomethyl group, a difluoromethyl group, a dichloromethyl group, a dibromomethyl group, a trifluoromethyl group, a trichloromethyl group, a tribromomethyl group, a 2,2,2-trifluoroethyl group, or a 2,2,2-trichloroethyl group; a hydroxy C1 to C6 alkyl group such as a hydroxymethyl group or a 2-hydroxyethyl group; a C1 to C6 alkoxy C1 to C6 alkyl group such as a methoxymethyl group, an ethoxymethyl group, a methoxyethyl group, an ethoxyethyl group, a methoxy-n-propyl group, a n-propoxymethyl group, an i-propoxyethyl group, a s-butoxymethyl group, or a t-butoxyethyl group; an amino C1 to C6 alkyl group such as an amino methyl group or an aminoethyl group; and a C1 to C6 alkylamino C1 to C6 alkyl group such as a methylaminomethyl group, an ethylaminomethyl group, a dimethylaminomethyl group or a diethylaminomethyl group.

Examples of the "alkenyl group having 2 to 6 carbon atoms" in G² include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group.

Examples of the "alkenyl group which is substituted with G¹ and has 2 to 6 carbon atoms" in G² include a haloalkenyl group having 2 to 6 carbon atoms such as a 2-chloro-1-propenyl group or a 2-fluoro-1-butenyl group; a hydroxy C2 to C6 alkenyl group such as a 2-hydroxy-1-propenyl group or a 2-hydroxy-1-butenyl group; a C1 to C6 alkoxy C2 to C6 alkenyl group such as a 2-methoxy-1-propenyl group or a 2-methoxy-1-butenyl group; an amino C2 to C6 alkenyl group such as a 2-amino-1-propenyl group or a 2-amino-1-butenyl group; and a C1 to C6 alkylamino C2 to C6 alkenyl group such as a 2-methylamino-1-propenyl group or a 2-dimethylamino-1-butenyl group.

Examples of the "alkynyl group having 2 to 6 carbon atoms" in G² include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group.

Examples of the "alkynyl group which is substituted with G¹ and has 2 to 6 carbon atoms" in G² include a haloalkynyl group having 2 to 6 carbon atoms such as a 4,4-dichloro-1-butynyl group, a 4-fluoro-1-pentynyl group, or a 5-bromo-2-pentynyl group; a hydroxy C2 to C6 alkynyl group such as a 4-hydroxy-1-pentynyl group or a 5-hydroxy-2-pentynyl group; a C1 to C6 alkoxy C2 to C6 alkynyl group such as a 4-methoxy-1-pentynyl group or a 5-methoxy-2-pentynyl group; an amino C2 to C6 alkynyl group such as a 4-amino-1-pentynyl group or a 5-amino-2-pentynyl group; and a C1 to C6 alkylamino C2 to C6 alkynyl group such as a 4-methylamino-1-pentynyl group or a 5-dimethylamino-2-pentynyl group.

Examples of the "cycloalkyl group having 3 to 8 carbon atoms" in G² include a cyclopropyl group, a cyclobutyl group, a cyclopentyl group, a cyclohexyl group, and a cycloheptyl group.

Examples of the "cycloalkyl group which is substituted with G¹ and has 3 to 8 carbon atoms" in G² include a halo C3 to C8 cycloalkyl group such as a 1-chloro-cyclopropyl group, a 1-chloro-cyclobutyl group, or a 3,3,4,4-tetrafluoro-cyclopentyl group; a hydroxy C3 to C8 cycloalkyl group such as a 1-hydroxy-cyclopropyl group or a 1-hydroxy-cyclobutyl group; a C1 to C6 alkoxy C3 to C8 cycloalkyl group such as a 1-methoxy-cyclopropyl group, a 1-methoxy-cyclobutyl group, or a 3,4-dimethoxy-cyclopentyl group; an amino C3 to C8 cycloalkyl group such as a 1-amino-cyclopropyl group, a 1-amino-cyclobutyl group, or a 3-amino-cyclopentyl group; and a C1 to C6 alkylamino C3 to C8 cycloalkyl group such as a 1-methylamino-cyclopropyl group, a 1-dimethylamino-cyclobutyl group, or a 3-dimethylamino-cyclopentyl group.

Examples of the "alkylidene group having 1 to 6 carbon atoms" in G² include a methylidene group, an ethylidene group, or a propylidene group.

Examples of the "alkylidene group which is substituted with G¹ and has 1 to 6 carbon atoms" in G² include a haloalkylidene group having 1 to 6 carbon atoms such as a 1-chloro-methylidene group, a 2-chloro-ethylidene group, or a 3,3,3-trifluoro-propylidene group; a hydroxy C1 to C6 alkylidene group such as a 1-hydroxy-methylidene group, a 2-hydroxy-ethylidene group, or a 3-hydroxy-propylidene group; a C1 to C6 alkoxy C1 to C6 alkylidene group such as a 1-methoxy-methylidene group, a 2-methoxy-ethylidene group, or a 3-methoxy-propylidene group; an amino C1 to C6 alkylidene group such as a 1-amino-methylidene group, a 2-amino-ethylidene group, or a 3-amino-propylidene group; and a C1 to C6 alkylamino C1 to C6 alkylidene group such as a 1-methylamino-methylidene group, a 2-dimethylamino-ethylidene group, or a 3-ethylamino-propylidene group.

Examples of the "alkylcarbonyl group having 1 to 6 carbon atoms" in G² include an acetyl group and a propionyl group.

Examples of the "alkylcarbonyl group which is substituted with G¹ and has 1 to 6 carbon atoms" in G² include a haloalkylcarbonyl group having 1 to 6 carbon atoms such as a chloroacetyl group, a trifluoroacetyl group, or a trichloroacetyl group; a hydroxy C1 to C6 alkylcarbonyl group such as a hydroxyacetyl group or a 2-hydroxypropionyl group; a C1 to C6 alkoxy C1 to C6 alkylcarbonyl group such as a methoxyacetyl group, an ethoxyacetyl group, a methoxypropionyl group, an ethoxypropionyl group, an ethoxyacetyl group, an ethoxypropionyl group, a n-propoxyacetyl group, an i-propoxypropionyl group, a s-butoxyacetyl group, or a t-butoxypropionyl group; an amino C1 to C6 alkylcarbonyl group such as an aminoacetyl group or an aminopropionyl group; and a C1 to C6 alkylamino C1 to C6 alkylcarbonyl group such as a methylaminoacetyl group, an ethylaminoacetyl group, a dimethylaminoacetyl group, or a diethylaminoacetyl group.

Examples of the "cycloalkylcarbonyl group having 3 to 8 carbon atoms" in G² include a cyclopropylcarbonyl group, a cyclobutylcarbonyl group, and a cyclopentylcarbonyl group.

Examples of the "cycloalkylcarbonyl group which is substituted with G¹ and has 3 to 8 carbon atoms" in G² include a halo C3 to C8 cycloalkylcarbonyl group such as a 1-chloro-cyclopropylcarbonyl group, a 1-chloro-cyclobutylcarbonyl group, or a 3,3,4,4-tetrafluoro-cyclopentylcarbonyl group; a hydroxy C3 to C8 cycloalkylcarbonyl group such as a 1-hydroxy-cyclopropylcarbonyl group or a 1-hydroxy-cyclobutylcarbonyl group; a C1 to C6 alkoxy C3 to C8 cycloalkylcarbonyl group such as a 1-methoxy-cyclopropylcarbonyl group, a 1-methoxy-cyclobutylcarbonyl group, or a 3,4-dimethoxy-cyclopentylcarbonyl group; an amino C3 to C8 cycloalkylcarbonyl group such as a 1-amino-cyclopropylcarbonyl group, a 1-amino-cyclobutylcarbonyl group, or a 3-amino-cyclopentylcarbonyl group; and a C1 to C6 alkylamino C3 to C8 cycloalkylcarbonyl group such as a 1-methylamino-cyclopropylcarbonyl group, a 1-dimethylamino-cyclobutylcarbonyl group, or a 3-dimethylamino-cyclopentylcarbonyl group.

Examples of the "alkoxycarbonyl group having 1 to 6 carbon atoms" in G² include a methoxycarbonyl group, an ethoxycarbonyl group, a n-propoxycarbonyl group, and an i-propoxycarbonyl group.

Examples of the "alkoxycarbonyl group which is substituted with G¹ and has 1 to 6 carbon atoms" in G² include a haloalkoxycarbonyl group having 1 to 6 carbon atoms such as a fluoromethoxycarbonyl group, a chloromethoxycarbonyl group, a bromomethoxycarbonyl group, a difluoromethoxycarbonyl group, a dichloromethoxycarbonyl group, a dibromomethoxycarbonyl group, a trifluoromethoxycarbonyl group, a trichloromethoxycarbonyl group, a tribromomethoxycarbonyl group, a 2,2,2-trifluoroethoxycarbonyl group, or a 2,2,2-trichloroethoxycarbonyl group; a hydroxy C1 to C6 alkoxycarbonyl group such as a hydroxymethoxycarbonyl group or a 2-hydroxyethoxycarbonyl group; a C1 to C6 alkoxy C1 to C6 alkoxycarbonyl group such as a methoxymethoxycarbonyl group, an ethoxymethoxycarbonyl group, a methoxyethoxycarbonyl group, an ethoxyethoxycarbonyl group, a methoxy-n-propoxycarbonyl group, a n-propoxymethoxycarbonyl group, an i-propoxyethoxycarbonyl group, a s-butoxymethoxycarbonyl group, or a t-butoxyethoxycarbonyl group; an amino C1 to C6 alkoxycarbonyl group such as an aminomethoxycarbonyl group or an aminoethoxycarbonyl group; and a C1 to C6 alkylamino C1 to C6 alkoxycarbonyl group such as a methylamino methoxycarbonyl group, an ethylamino methoxycarbonyl group, a dimethylamino methoxycarbonyl group, or a diethylamino methoxycarbonyl group.

Examples of the "alkylsulfonyl group having 1 to 6 carbon atoms" in G² include a methylsulfonyl group, an ethylsulfonyl group, and a t-butylsulfonyl group.

Examples of the "alkylsulfonyl group which is substituted with G¹ and has 1 to 6 carbon atoms" in G² include a haloalkylsulfonyl group having 1 to 6 carbon atoms such as a fluoromethylsulfonyl group, a chloromethylsulfonyl group, a bromomethylsulfonyl group, a difluoromethylsulfonyl group, a dichloromethylsulfonyl group, a dibromomethylsulfonyl group, a trifluoromethylsulfonyl group, a trichloromethylsulfonyl group, a tribromomethylsulfonyl group, a 2,2,2-trifluoroethylsulfonyl group, or a 2,2,2-trichloroethylsulfonyl group; a hydroxy C 1 to C6 alkylsulfonyl group such as a hydroxymethylsulfonyl group or a 2-hydroxyethylsulfonyl group; a C1 to C6 alkoxy C1 to C6 alkylsulfonyl group such as a methoxymethylsulfonyl group, an ethoxymethylsulfonyl group, a methoxyethylsulfonyl group, an ethoxyethylsulfonyl group, a methoxy-n-propylsulfonyl group, a n-propoxymethylsulfonyl group, an i-propoxyethylsulfonyl group, a s-butoxymethylsulfonyl group, or a t-butoxyethyl group; an amino C1 to C6 alkylsulfonyl group such as an aminomethylsulfonyl group or an aminoethylsulfonyl group; and a C1 to C6 alkylamino C1 to C6 alkylsulfonyl group such as a methylamino methylsulfonyl group, an ethylamino methylsulfonyl group, a dimethylamino methylsulfonyl group, or a diethylamino methylsulfonyl group.

As the "alkyl group having 1 to 6 carbon atoms," the "alkyl group which is substituted with G¹ and has 1 to 6 carbon atoms," the "alkenyl group having 2 to 6 carbon atoms," the "alkenyl group which is substituted with G¹ and has 2 to 6 carbon atoms," the "alkynyl group having 2 to 6 carbon atoms," the "alkynyl group which is substituted with G¹ and has 2 to 6 carbon atoms," the "cycloalkyl group having 3 to 8 carbon atoms," and the "cycloalkyl group which is substituted with G¹ and has 3 to 8 carbon atoms" in R^{1a} and R^{1b} the same groups as described above in G² are exemplary examples.

As the "alkoxy group having 1 to 6 carbon atoms" in R^{1a} and R^{1b}, the same groups as described above in the "alkoxy group having 1 to 6 carbon atoms" in G¹ are exemplary examples.

Examples of the "alkoxy group which is substituted with G¹ and has 1 to 6 carbon atoms" in R^{1a} and R^{1b} include a haloalkoxy group having 1 to 6 carbon atoms such as a fluoromethoxy group, a chloromethoxy group, a bromomethoxy group, a difluoromethoxy group, a dichloromethoxy group, a dibromomethoxy group, a trifluoromethoxy group, a trichloromethoxy group, a tribromomethoxy group, a 2,2,2-trifluoroethoxy group, or a 2,2,2-trichloroethoxy group; a hydroxy C1 to C6 alkoxy group such as a hydroxymethoxy group or a 2-hydroxyethoxy group; a C1 to C6 alkoxy C1 to C6 alkoxy group such as a methoxymethoxy group, an ethoxymethoxy group, a methoxyethoxy group, an ethoxyethoxy group, a methoxy-n-propoxy group, a n-propoxymethoxy group, an i-propoxyethoxy group, a s-butoxymethoxy group, or a t-butoxyethoxy group; an amino C1 to C6 alkoxy group such as an aminomethoxy group or an aminoethoxy group; and a C1 to C6 alkylamino C1 to C6 alkoxy group such as a methylaminomethoxy group, an ethylaminomethoxy group, a dimethylaminomethoxy group, or a diethylaminomethoxy group.

Examples of the "alkenyloxy group having 2 to 6 carbon atoms" in R^{1a} and R^{1b} include an ethenyloxy group, a 1-methyl-2-propenyloxy group, and a 2-methyl-1-propenyloxy group.

Examples of the "alkenyloxy group which is substituted with G¹ and has 2 to 6 carbon atoms" in R^{1a} and R^{1b} include a haloalkenyloxy group having 2 to 6 carbon atoms such as a 2-chloro-1-propenyloxy group or a 2-fluoro-1-butenyloxy group; a hydroxy C2 to C6 alkenyloxy group such as a 2-hydroxy-1-propenyloxy group or a 2-hydroxy-1-butenyloxy group; a C1 to C6 alkoxy C2 to C6 alkenyloxy group such as a 2-methoxy-1-propenyloxy group or a 2-methoxy-1-butenyl group; an amino C2 to C6 alkenyloxy group such as a 2-amino-1-propenyloxy group or a 2-amino-1-butenyloxy group; and a C1 to C6 alkylamino C2 to C6 alkenyloxy group such as a 2-methylamino-1-propenyloxy group or a 2-dimethylamino-1-butenyloxy group.

Examples of the "alkynyloxy group having 2 to 6 carbon atoms" in R^{1a} and R^{1b} include an ethynyloxy group, a propargyloxy group, a 1-methylpropargyloxy group, and a 2-butynyloxy group.

Examples of the "alkynyloxy group which is substituted with G¹ and has 2 to 6 carbon atoms in R^{1a} and R^{1b} include a haloalkynyloxy group having 2 to 6 carbon atoms such as a 4,4-dichloro-1-butynyloxy group, a 4-fluoro-1-pentynyloxy group, or a 5-bromo-2-pentynyloxy group; a hydroxy C2 to C6 alkynyloxy group such as a 4-hydroxy-1-pentynyloxy group or a 5-hydroxy-2-pentynyloxy group; a C1 to C6 alkoxy C2 to C6 alkynyloxy group such as a 4-methoxy-1-pentynyloxy group or a 5-methoxy-2-pentynyloxy group; an amino C2 to C6 alkynyloxy group such as a 4-amino-1-pentynyloxy group or a 5-amino-2-pentynyloxy group; a C1 to C6 alkylamino C2 to C6 alkynyloxy group such as a 4-methylamino-1-pentynyloxy group or a 5-dimethylamino-2-pentynyloxy group.

Examples of the "amino group substituted with one G²" in R^{1a} and R^{1b} include an "alkylamino group having 1 to 6 carbon atoms" such as a methylamino group, an ethylamino group, a n-propylamino group, or a n-butylamino group; an "alkenylamino group having 2 to 6 carbon atoms" such as a vinylamino group, a 1-propenylamino group, a 2-propenylamino group, or a 1-butenylamino group; an "alkynylamino group having 2 to 6 carbon atoms" such as an ethynylamino group, a 1-propynylamino group, a 2-propynylamino group, a 1-butynylamino group, or a 2-butynylamino group; a "cycloalkylamino group having 3 to 8 carbon atoms" such as a cyclopropylamino group, a cylobutylamino group, a cyclopentylamino group, or a cyclohexylamino group; an "alkylideneamino group having 1 to 6 carbon atoms" such as a methylene amino group, an ethylideneamino group, or a propylideneamino group; an "alkylcarbonylamino group having 1 to 6 carbon atoms" such as an acetylamino group or a propionylamino group; a "cycloalkylcarbonylamino group having 3 to 8 carbon atoms" such as a cyclopropylcarbonylamino group, a cyclobutylcarbonylamino group, or a cyclopentylcarbonylamino group; an "alkoxycarbonylamino group having 1 to 6 carbon atoms" such as a methoxycarbonylamino group, an ethoxycarbonylamino group, a n-propoxycarbonylamino group, or an i-propoxycarbonylamino group; and an "alkylsulfonylamino group having 1 to 6 carbon atoms" such as a methylsulfonylamino group, an ethylsulfonylamino group, or a t-butylsulfonylamino group.

Examples of the "amino group substituted with two G²s which are the same as or different from each other" in R^{1a} and R^{1b} include a "di-C1 to C6 alkylamino group" such as a dimethylamino group, an ethyl-methyl-amino group, a n-propyl-methyl-amino group, or a n-butyl-methyl-amino group; a "C2 to C6 alkenyl-(C1 to C6 alkyl)amino group" such as a vinyl-methyl-amino group, a 1-propenyl-methyl-amino group, a 2-propenyl-methyl-amino group, or a 1-butenyl-methyl-amino group; a "C2 to C6 alkynyl-(C1 to C6 alkyl)amino group" such as an ethynyl-methyl-amino group, a 1-propynyl-methyl-amino group, a 2-propyl-methyl-amino group, a 1-butynyl-methyl-amino group, or a 2-butynyl-methyl-amino group; a "C3 to C8 cycloalkyl-(C1-C6 alkyl)amino group" such as a cyclopropyl-methyl-amino group, a cyclobutyl-methyl-amino group, a cyclopentyl-methyl-amino group, or a cyclohexyl-methyl-amino group; a "C1 to C6 alkylcarbonyl-(C1 to C6 alkyl)amino group" such as an acetyl-methyl-amino group, or a propionyl-methyl-amino group; a "C3 to C8 cycloalkylcarbonyl-(C1 to C6 alkyl)amino group" such as a cyclopropylcarbonyl-methyl-amino group, a cyclobutylcarbonyl-methyl-amino group, or a cyclopentylcarbonyl-methyl-amino group; a "C1 to C6 alkoxycarbonyl-(C1 to C6 alkyl)amino group" such as a methoxycarbonyl-methyl-amino group, an ethoxycarbonyl-methyl-amino group, a n-propoxycarbonyl-methyl-amino group, or an i-propoxycarbonyl-methyl-amino group; and a "C1 to C6 alkylsulfonyl-(C1 to C6 alkyl)amino group such as a methylsulfonyl-methyl-amino group, an ethylsulfonyl-methyl-amino group, or a t-butylsulfonyl-methyl-amino group.

In addition, examples of the "the ring formed by an amino group being substituted with two G²s and the two G²s being bonded to each other" include saturated heterocyclic groups such as an aziridine ring, a pyrrolidine ring, a piperidine ring, a piperazine ring, and a morpholine ring.

As the "alkyl group having 1 to 6 carbon atoms" in R^{1c}, the same groups as described above in G² are exemplary examples.

### [R²]

R² represents an amino group, an amino group substituted with one G², an amino group substituted with two G²s which are the same as or different from each other, a hydroxyl group, an alkoxy group having 1 to 6 carbon atoms, an alkylcarbonyloxy group having 1 to 6 carbon atoms, an alkoxycarbonyloxy group having 1 to 6 carbon atoms, a cyano group, or an alkyl group which is substituted with G³ and has 1 to 6 carbon atoms. a represents an integer of 1 to 4, and when a represents 2 or greater, R²s may be the same as or different from each other.

G² in R² has the same definition as that for G² in R^{1a} and R^{1b} and examples thereof are the same as those exemplified above in the description of R^{1a} and R^{1b}.

As the "amino group substituted with one G²" and the "amino group substituted with two G²s which are the same as or different from each other" in R², the same as groups described above in R^{1a} are exemplary examples. As the "alkoxy group having 1 to 6 carbon atoms" in R², the same as groups described above in G¹ are exemplary examples.

Examples of the "alkylcarbonyloxy group having 1 to 6 carbon atoms" in R² include an acetyloxy group and a propionyloxy group.

Examples of the "alkoxycarbonyloxy group having 1 to 6 carbon atoms" in R² include a methoxycarbonyloxy group and an ethoxycarbonyloxy group.

G³ in R² represents an amino group, an amino group substituted with one G², an amino group substituted with two G²s which are the same as or different from each other, a hydroxyl group, an alkoxy group having 1 to 6 carbon atoms, an alkylcarbonyloxy group having 1 to 6 carbon atoms, an alkoxycarbonyloxy group having 1 to 6 carbon atoms, or a cyano group. G² in G³ has the same definition as that described above and examples thereof are the same as those described above.

As the "amino group substituted with one G²" and the "amino group substituted with two G²s which are the same as or different from each other" in G³, the same as groups described in R^{1a} are exemplary examples. As the "alkoxy group having 1 to 6 carbon atoms" in G³, the same as groups described in G¹ are exemplary examples. As the "alkylcarbonyloxy group having 1 to 6 carbon atoms" and the "alkoxycarbonyloxy group having 1 to 6 carbon atoms," the same as groups described in R² are exemplary examples.

Examples of the "alkyl group which is substituted with G³ and has 1 to 6 carbon atoms" in R² include an alkyl group which is substituted with an amino group and has 1 to 6 carbon atoms, an alkyl group which is substituted with an amino group substituted with one G² and has 1 to 6 carbon atoms, an alkyl group which is substituted with an amino group substituted with two G²s which are the same as or different from each other and has 1 to 6 carbon atoms; an alkyl group which is substituted with a hydroxyl group and has 1 to 6 carbon atoms, an alkyl group which is substituted with an alkoxy group having 1 to 6 carbon atoms and has 1 to 6 carbon atoms, an alkyl group which is substituted with an alkylcarbonyloxy group having 1 to 6 carbon atoms and has 1 to 6 carbon atoms, an alkyl group which is substituted with an alkoxycarbonyloxy group having 1 to 6 carbon atoms and has 1 to 6 carbon atoms, and an alkyl group which is substituted with a cyano group and has 1 to 6 carbon atoms.

Examples of the "alkyl group which is substituted with an amino group and has 1 to 6 carbon atoms" in R² include an aminomethyl group and an aminoethyl group.

Examples of the "alkyl group which is substituted with an amino group substituted with one G² and has 1 to 6 carbon atoms" in R² include a "C1 to C6 alkylamino C1 to C6 alkyl group" such as a methylaminomethyl group, an ethylaminomethyl group, a n-propylaminomethyl group, or a n-butylaminomethyl group; a "C2 to C6 alkenylamino C1 to C6 alkyl group" such as a vinylaminomethyl group, a 1-propenylaminomethyl group, a 2-propenylaminomethyl group, or a 1-butenylaminomethyl group; a "C2 to C6 alkynylamino C1 to C6 alkyl group" such as an ethynylaminomethyl group, a 1-propynylaminomethyl group, a 2-propylaminomethyl group, a 1-butynylaminomethyl group, or a 2-butynylaminomethyl group; a "C3 to C8 cycloalkylamino C1 to C6 alkyl group" such as a cyclopropylaminomethyl group, a cyclobutylaminomethyl group, a cyclopentylamino group, or a cyclohexylaminomethyl group; a "C1 to C6 alkylideneamino C1 to C6 alkyl group" such as a methylideneaminomethyl group, an ethylideneaminomethyl group, or a propylideneaminomethyl group; a "C1 to C6 alkylcarbonylamino C1 to C6 alkyl group" such as an acetylaminomethyl group or a propionylaminomethyl group; a "C3 to C8 cycloalkylcarbonylamino C1 to C6 alkyl group" such as a cyclopropylcarbonylaminomethyl group, a cyclobutylcarbonylaminomethyl group, or a cyclopentylcarbonylaminomethyl group; a "C1 to C6 alkoxycarbonylamino C1 to C6 alkyl group" such as a methoxycarbonylaminomethyl group, an ethoxycarbonylaminomethyl group, a n-propoxycarbonylaminomethyl group, or an i-propoxycarbonylaminomethyl group; and a "C1 to C6 alkylsulfonylamino C1 to C6 alkyl group" such as a methylsulfonylaminomethyl group, an ethylsulfonylaminomethyl group, or a t-butylsulfonylaminomethyl group.

Examples of the "alkyl group which is substituted with an amino group substituted with two G²s which are the same as or different from each other and has 1 to 6 carbon atoms" in R² include a "di-C1 to C6 alkylamino C1 to C6 alkyl group" such as a dimethylaminomethyl group, an ethyl-methyl-aminomethyl group, a n-propyl-methyl-aminomethyl group, or a n-butyl-methyl-aminomethyl group; a "C2 to C6 alkenyl-(C1 to C6 alkyl) amino C1 to C6 alkyl group" such as a vinyl-methyl-aminomethyl group, a 1-propenyl-methyl-aminomethyl group, a 2-propenyl-methyl-aminomethyl group, or a 1-butenyl-methyl-aminomethyl group; a "C2 to C6 alkynyl-(C1 to C6 alkyl) amino C1 to C6 alkyl group" such as an ethynyl-methyl-aminomethyl group, a 1-propynyl-methyl-aminomethyl group, a 2-propynyl-mehyl-aminomethyl group, a 1-butynyl-methyl-aminomethyl group, or a 2-butynyl-methyl-aminomethyl group; a "C3 to C8 cycloalkyl-(C1 to C6 alkyl) amino C1 to C6 alkyl group" such as a cyclopropyl-methyl-aminomethyl group, a cyclobutyl-methyl-aminomethyl group, a cyclopentyl-methyl-aminomethyl group, or a cyclohexyl-methyl-aminomethyl group; a "C1 to C6 alkylcarbonyl-(C1 to C6 alkyl) amino C1 to C6 alkyl group" such as an acetyl-methyl-aminomethyl group or a propionyl-methyl-aminomethyl group; a "C3 to C8 cycloalkylcarbonyl-(C1 to C6 alkyl) amino C1 to C6 alkyl group" such as a cyclopropylcarbonyl-methyl-aminomethyl group, a cyclobutylcarbonyl-methyl-aminomethyl group, or a cyclopentylcarbonyl-methyl-aminomethyl group; a "C1 to C6 alkoxycarbonyl-(C1 to C6 alkyl) amino C1 to C6 alkyl group" such as a methoxycarbonyl-methyl-aminomethyl group, an ethoxycarbonyl-methyl-aminomethyl group, a n-propoxycarbonyl-methyl-aminomethyl group, or an i-propoxycarbonyl-methyl-aminomethyl group; and a "C1 to C6 alkylsulfonyl-(C1 to C6 alkyl) amino C1 to C6 alkyl group" such as a methylsulfonyl-methyl-aminomethyl group, an ethylsulfonyl-methyl-aminomethyl group, or a t-butylsulfonyl-methyl-aminomethyl group.

Examples of the "alkyl group which is substituted with a hydroxyl group and has 1 to 6 carbon atoms" in R² include a "hydroxy C1 to C6 alkyl group" such as a hydroxymethyl group or a 2-hydroxyethyl group.

Examples of the "alkyl group which is substituted with an alkoxy group having 1 to 6 carbon atoms and has 1 to 6 carbon atoms" in R² include a "C1 to C6 alkoxy C1 to C6 alkyl group" such as a methoxymethyl group, an ethoxymethyl group, a methoxyethyl group, an ethoxyethyl group, a methoxy-n-propyl group, a n-propoxymethyl group, an i-propoxyethyl group, a s-butoxymethyl group, or a t-butoxyethyl group.

Examples of the "alkyl group which is substituted with an alkylcarbonyloxy group having 1 to 6 carbon atoms and has 1 to 6 carbon atoms" in R² include a "C1 to C6 alkylcarbonyloxy C1 to C6 alkyl group" such as an acetyloxymethyl group or a propionyloxymethyl group.

Examples of the "alkyl group which is substituted with an alkoxycarbonyloxy group having 1 to 6 carbon atoms and has 1 to 6 carbon atoms" in R² include a "C1 to C6 alkoxycarbonyloxy C1 to C6 alkyl group" such as a methoxycarbonyloxymethyl group or an ethoxycarbonyloxymethyl group.

Examples of the "alkyl group which is substituted with a cyano group and has 1 to 6 carbon atoms" in R² include a "cyano C1 to C6 alkyl group" such as a cyanomethyl group or a 2-cyanoethyl group.

### [R³]

R³ represents a halogen atom or an organic group other than G³. G³ in R³ has the same definition as that described in R² and examples thereof are the same as those exemplified in the description above.

b indicates the number of R³s and represents an integer of 0 to 3, and when b represents 2 or greater, R³s may be the same as or different from each other provided that, a relationship of "a + b ≤ 4" is satisfied.

Examples of the "organic group other than G³" in R³ include an arylamino group such as a benzylamino group, a phenylamino group, or a phenylethylamino group, preferably an arylamino group having 6 to 10 carbon atoms; a nitro group; a formyl group; an alkyl group having 1 to 6 carbon atoms such as a methyl group, an ethyl group, a n-propyl group, an isopropyl group, a n-butyl group, a s-butyl group, an isobutyl group, a t-butyl group, a n-pentyl group, or a n-hexyl group; an alkenyl group having 2 to 6 carbon atoms such as a vinyl group or an allyl group; an alkynyl group having 2 to 6 carbon atoms such as an ethynyl group, a 1-propynyl group or a propargyl group; an alkenyloxy group having 2 to 6 carbon atoms such as a vinyloxy group or an allyloxy group; an alkynyloxy group having 2 to 6 carbon atoms such as an ethynyloxy group or a propargyloxy group; an aryloxy group such as a benzyloxy group or a phenoxy group, preferably an aryloxy group having 6 to 10 carbon atoms; a haloalkyl group having 1 to 6 carbon atoms such as a chloromethyl group, a fluoromethyl group, a bromomethyl group, a dichloromethyl group, a difluoromethyl group, a dibromomethyl group, a trichloromethyl group, a trifluoromethyl group, a bromodifluoromethyl group, a 1,1,1-trifluoroethyl group, a 1-chloroethyl group, a 2-chloroethyl group, a 1-bromoethyl group, or a pentafluoroethyl group; a haloalkoxy group having 1 to 6 carbon atoms such as a fluoromethoxy group, a chloromethoxy group, a bromomethoxy group, a difluoromethoxy group, a dichloromethoxy group, a dibromomethoxy group, a trifluoromethoxy group, a trichloromethoxy group, a tribromomethoxy group, a trifluoroethoxy group, a pentafluoroethoxy group, or a heptafluoro n-propoxy group; an alkylthiocarbonyl group having 1 to 6 carbon atoms such as a methylthiocarbonyl group, an ethylthiocarbonyl group, a n-propylthiocarbonyl group, an isopropylthiocarbonyl group, a n-butylthiocarbonyl group, an isobutylthiocarbonyl group, a s-butylthiocarbonyl group, or a t-butylthiocarbonyl group; an alkoxycarbonyl group having 1 to 6 carbon atoms such as a methoxycarbonyl group, an ethoxycarbonyl group, a n-propoxycarbonyl group, an isopropoxycarbonyl group, a n-butoxycarbonyl group, or a t-butoxycarbonyl group; an aryl group such as a phenyl group, a 1-naphthyl group, or a 2-naphthyl group, preferably an aryl group having 6 to 10 carbon atoms; a 5-membered heteroaryl group such as a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, or a tetrazolyl group; a 6-membered heteroaryl group such as a pyridyl group, a pyrazinyl group, a pyrimidinyl group, a pyridazinyl group, or a triazinyl group; a saturated heterocyclic group such as an aziridinyl group, an epoxy group, a pyrrolidinyl group, a tetrahydrofuranyl group, a piperidyl group, a piperazinyl group, or a morpholinyl group; an alkylthio group having 1 to 6 carbon atoms such as a methylthio group, an ethylthio group, or a t-butylthio group; an alkylsulfonyl group such as a methylsulfonyl group, an ethylsulfonyl group, or a t-butylsulfonyl group; an alkenylsulfonyl group having 2 to 6 carbon atoms such as an allylsulfonyl group; an alkynylsulfonyl group such as a propargylsulfonyl group, preferably an alkynylsulfonyl group having 2 to 6 carbon atoms; an arylsulfonyl group such as a phenylsulfonyl group, and preferably an arylsulfonyl group having 6 to 10 carbon atoms.

### [R⁴]

R⁴ represents a cyano group or an alkyl group which is substituted with G³ and has 1 to 6 carbon atoms. c represents an integer of 0 to 3, and when c represents 2 or more, R⁴s may be the same as or different from each other.

G³ in R⁴ has the same definition as that described in R² and examples thereof are the same as those exemplified in the description above.

Examples of the "alkyl group which is substituted with G³ and has 1 to 6 carbon atoms" in R⁴, the same as groups described in R² are exemplary examples.

### [R⁵]

R⁵ represents a halogen atom or an organic group other than G³. d indicates the number of R⁵S and represents an integer of 0 to 3, and when d represents 2 or more, R⁵s may be the same as or different from each other, provided that, a relationship of "c + d ≤ 3" is satisfied.

Examples of the organic groups other than G³ in R⁵ are the same as those described as the organic groups other than G³ in R³.

### [R⁶]

R⁶ represents an alkyl group having 1 to 6 carbon atoms.
n represents an integer of 0 to 4, and when n represents 2 or greater, R⁶s may be the same as or different from each other and two R⁶s may be bonded to each other to form an alkylene group having 2 to 6 carbon atoms.
m represents an integer of 0 to 3, and when m represents 2 or greater, R⁶s may be the same as or different from each other and two R⁶s may be bonded to each other to form an alkylene group having 2 to 6 carbon atoms.

As the "alkyl group having 1 to 6 carbon atoms" in R⁶, the same as groups described in G² are exemplary examples.

As the "alkylene group having 2 to 6 carbon atoms" which is formed by R⁶s being bonded to each other, an ethylene group or a propylene group is an exemplary example.

In the phenylimidazole derivative of the present invention, it is preferable that, in Formula (I), A represents a nitrogen atom, B¹ represents a group represented by N-COR^{1a}, and an imidazolyl group which is a substituent of the benzene ring is an imidazole-1-yl group. That is, it is preferable that the phenylimidazole derivative of the present invention is a phenylimidazole derivative represented by Formula (IA) or a salt thereof. 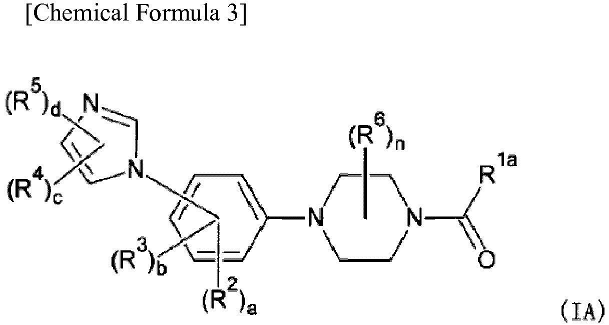 In Formula (IA), R^{1a}, R², R³, R⁴, R⁵, R⁶, a, b, c, d, and n are the same as those defined in Formula (I).

In the phenylimidazole derivative of the present invention, it is preferable that, in Formula (I), A represents a nitrogen atom, B¹ represents a group represented by N-COR^{1a}, an imidazolyl group which is a substituent of the benzene ring is an imidazole-1-yl group, and the imidazolyl group is in a meta-position with respect to a piperazine ring. That is, it is preferable that the phenylimidazole derivative of the present invention is a phenylimidazole derivative represented by Formula (IB) or a salt thereof. 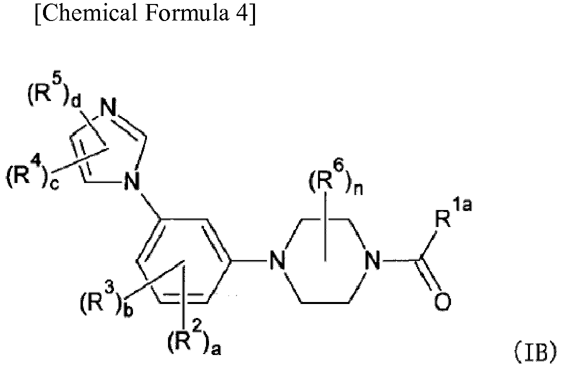 In Formula (IB), R^{1a}, R², R³, R⁴, R⁵, R⁶, a, b, c, d, and n are the same as those defined in Formula (I).

In the phenylimidazole derivative of the present invention, it is preferable that, in Formula (II), A represents a nitrogen atom, B² represents a group represented by NR^{1c}-COR^{1a}, and an imidazolyl group which is a substituent of the benzene ring is an imidazole-1-yl group. That is, it is preferable that the phenylimidazole derivative of the present invention is a phenylimidazole derivative represented by Formula (IIA) or a salt thereof. 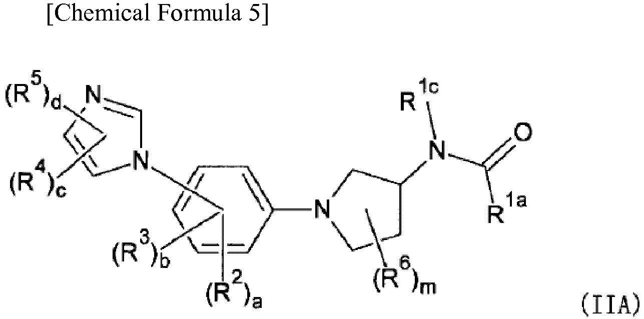 In Formula (IIA), R^{1a}, R^{1c}, R², R³, R⁴, R⁵, R⁶, a, b, c, d, and m are the same as those defined in Formula (II).

In the phenylimidazole derivative of the present invention, it is preferable that, in Formula (II), A represents a nitrogen atom, B² represents a group represented by NR^{1c}-COR^{1a}, an imidazolyl group which is a substituent of the benzene ring is an imidazole-1-yl group, and the imidazolyl group be in a meta-position with respect to a pyrrolidine ring. That is, it is preferable that the phenylimidazole derivative of the present invention is a phenylimidazole derivative represented by Formula (IIB) or a salt thereof. 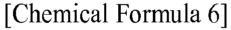 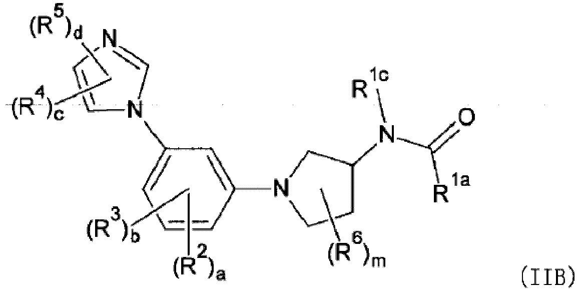 In Formula (IIB), R^{1a}, R^{1c}, R², R³, R⁴, R⁵, R⁶, a, b, c, d, and m have the same definitions as those described in Formula (II).

A salt of the phenylimidazole derivative is not particularly limited as long as the salt is pharmacologically acceptable. Examples of such a salt include a salt of an inorganic acid such as hydrochloric acid, sulfuric acid, nitric acid, or phosphoric acid; and a salt of an organic acid such as acetic acid, oxalic acid, mandelic acid, propionic acid, lactic acid, succinic acid, tartaric acid, citric acid, benzoic acid, salicylic acid, nicotinic acid, hepta gluconic acid, methane sulfonic acid, benzene sulfonic acid, or p-toluene sulfonic acid. A pharmacologically acceptable salt contains an acid addition salt formed of a free amino group of a polypeptide or an antibody molecule. These can be easily produced by a general chemically-synthesized method.

The phenylimidazole derivative of the present invention or a salt thereof includes a geometric isomer, an optical isomer, or a tautomer. In addition, the phenylimidazole derivative of the present invention or a salt thereof includes a hydrate or a solvate.

The phenylimidazole derivative of the present invention or a salt thereof includes crystal polymorph and a crystal polymorphic group (crystal polymorphic system). In addition, the crystal polymorphic group (crystal polymorphic system) indicates respective crystal forms and the entire process in respective steps in a case where the crystal form is changed due to the conditions and states (further, the present state also includes a formulated state) such as production, crystallization, or storage of these crystals.

A method of producing the phenylimidazole derivative of the present invention is not particularly limited. For example, the phenylimidazole derivative can be produced in the following manner.

### [Production method 1]

A phenylimidazole derivative (a compound represented by Formula (6), hereinafter, referred to as a compound (6)) in which, in Formula (I), A represents a nitrogen atom; B¹ represents a group represented by N-COR^{1a}; an imidazolyl group is an imidazole-1-yl group; and R² represents an amino group can be produced by the following method.

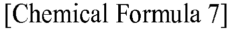

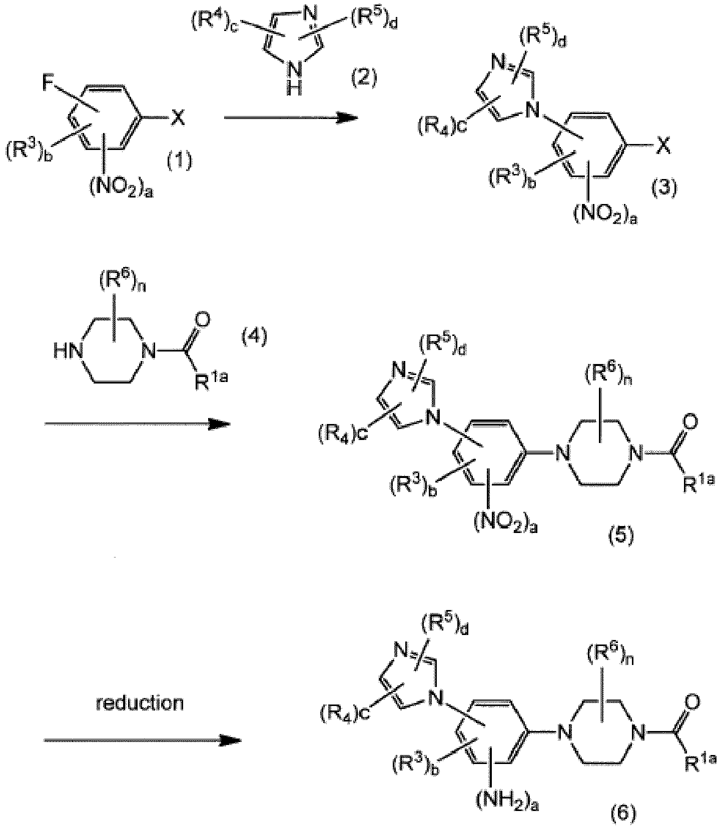

A compound represented by Formula (3) (a, b, c, d, R³, R⁴, and R⁵ in the formula are the same as those defined above in Formula (I). X represents a halogen atom. Hereinafter, this compound is referred to as a compound (3)) can be obtained by fusing a compound represented by Formula (1) (a, b, and R³ in the formula are the same as those defined above in Formula (I). X represents a halogen atom. Hereinafter, this compound is referred to as a compound (1)) and a compound represented by Formula (2) (c, d, R⁴, and R⁵ in the formula are the same as those defined above in Formula (I). Hereinafter, this compound is referred to as the compound (2)) in the presence of a base. The temperature at the time of a condensation reaction is generally in the range of 0°C to a boiling point of a solvent to be used.

In addition, a compound (a, b, c, d, n, R^{1a}, R³, R⁴, R⁵, and R⁶ in the formula are the same as those defined in Formula (I). Hereinafter, this compound is referred to as a compound (5)) represented by Formula (5) can be obtained by fusing the compound (3) and a compound (R^{1a}, R⁶, and n in the formula are the same as those described in Formula (I). Hereinafter, this compound is referred to as a compound (4)) represented by Formula (4) in the presence of a base. The temperature at the time of a condensation reaction is generally in the range of 0°C to a boiling point of a solvent to be used.

The base used for the condensation reaction of the compound (1) and the compound (2) and the condensation reaction of the compound (3) and the compound (4), are not particularly limited. Examples thereof include amines such as trimethylamine, pyridine, and 1,8-diazabicyclo[5.4.0]undec-7-ene (hereinafter, referred to as "DBU") and inorganic bases such as sodium bicarbonate, sodium carbonate, potassium carbonate, and sodium hydroxide.

The condensation reaction of the compound (1) and the compound (2) and the condensation reaction of the compound (3) and the compound (4) can be performed in an appropriate solvent. The solvent to be used is not particularly limited as long as the solvent is inactive at the time of a reaction. Examples thereof include alcohols such as methanol and ethanol; ethers such as diethyl ether, tetrahydrofuran, and 1,4-dioxane; hydrocarbons such as benzene, toluene, xylene, and cyclohexane; amides such as N,N-dimethylformamide; organic acids such as formic acid and acetic acid; esters such as ethyl acetate; and a mixed solvent formed of two or more of these.

Next, a compound (6) (a, b, c, d, n, R^{1a}, R³, R⁴, R⁵, and R⁶ in the formula are the same as those defined in Formula (I)) can be obtained by reducing the compound (5). The reduction reaction can be performed using a reducing agent or a hydrogenation catalyst. Examples of the reduction reaction include a method of performing reduction using hydrochloric acid and stannous chloride in an alcohol solvent such as methanol or ethanol; a method of performing reduction using acetic acid and iron in a mixed solvent of a ketone solvent such as acetone or methyl ethyl ketone and water; a method of performing reduction using ammonium chloride, ammonium acetate, ammonium formate, or acetic acid and zinc in alcohol or a mixed solvent of alcohol and water; and a method of performing a hydrogenation reaction using a known hydrogenation catalyst such as palladium carbon, palladium hydroxide, platinum dioxide, or Raney nickel in alcohols such as methanol and ethanol; ethers such as diethyl ether, tetrahydrofuran, and 1,4-dioxane; hydrocarbons such as benzene, toluene, xylene, and cyclohexane; amides such as N,N-dimethylformamide and N,N-dimethylacetamide; organic acids such as formic acid and acetic acid; esters such as ethyl acetate; or a mixed solvent formed of two or more of these. The temperature at the time of a reduction reaction is normally in the range of 0°C to a boiling point of a solvent to be used.

### [Production method 2]

Aphenylimidazole derivative (a compound represented by Formula (6b)) in which A in Formula (II) represents a nitrogen atom, B² represents a group represented by NR^{1c}-COR^{1a}, an imidazolyl group is an imidazole-1-yl group, and R² represents an amino group can be obtained in the same manner as in Production method 1, except that a compound (R^{1a}, R^{1c}, R⁶, and m in the formula are the same as those defined in Formula (II)) represented by Formula (4b) is used instead of the compound (4) used in Production method 1.

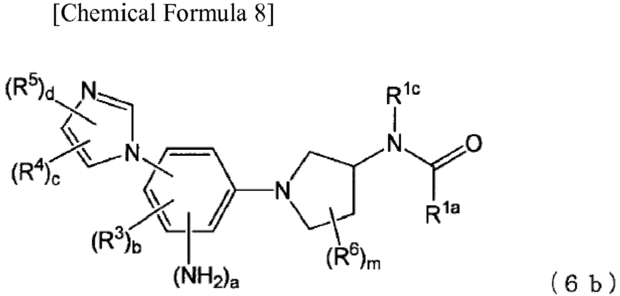

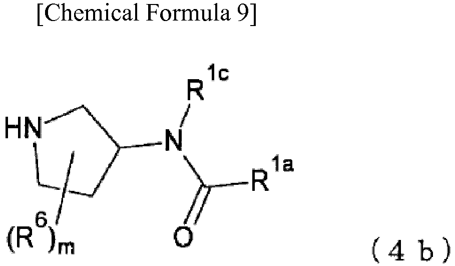

### [Production method 3]

Aphenylimidazole derivative (a compound represented by Formula (15), hereinafter, referred to as a compound (15)) in which A in Formula (I) represents a nitrogen atom, B¹ represents a group represented by N-COR^{1a}, an imidazolyl group is an imidazole-1-yl group, and R² represents an amino group can be produced by the following method.

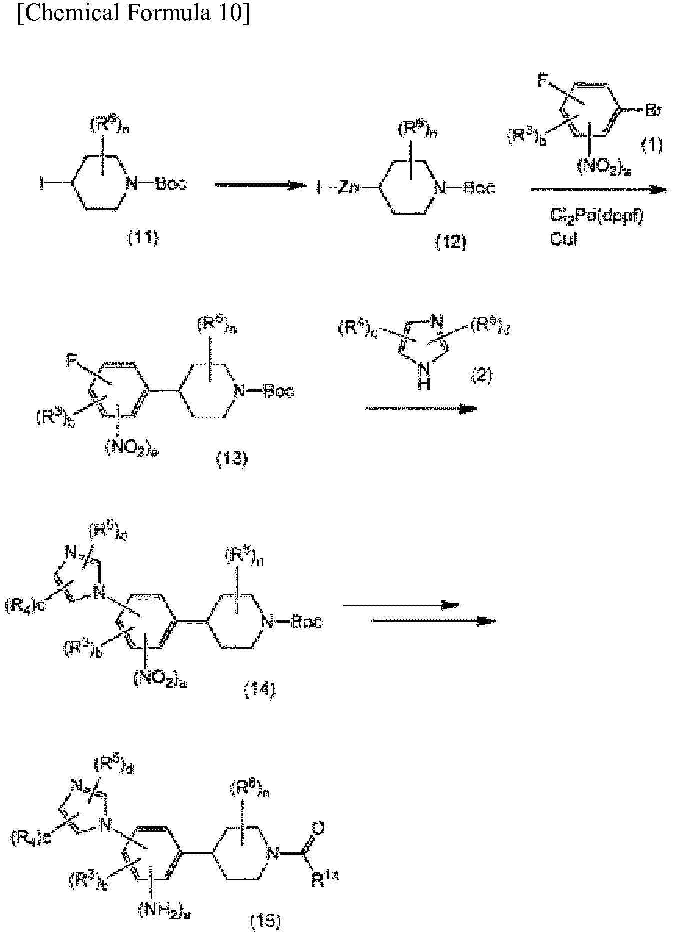

Zinc is inserted to a compound (R⁶ and n in the formula are the same as those defined above) represented by Formula (11) according to a method described in J. Org. Chem. 2004, 69, 5120 to induce a compound (R⁶ and n in the formula have the same definitions as those described above; hereinafter, referred to as a compound (12)) represented by Formula (12) .

Next, a compound (R⁶, n, a, b, and R³ are the same as those defined above, hereinafter, referred to as a compound (13)) represented by Formula (13) can be obtained by performing a coupling reaction of the compound (12) and the compound (1) using a palladium catalyst.

Examples of the palladium catalyst include bis(triphenylphosphine)palladium (II) dichloride [PdCl₂(PPh₃)₂], palladium acetate [Pd(OAc)₂], tetrakistriphenylphosphine palladium (0) [Pd(PPh₃)₂], palladium (II) chloride [PdCl₂], tris(dibenzylidene acetone)dipalladium (0) [Pd₂(dba)₃], Pd/C, palladium-alumina, and [1,1'-bis(diphenylphosphine)-ferrocene]palladium II dichlodride [PdCl₂(dppf)]. These catalysts can be used alone or in combination of two or more kinds thereof In addition, a copper catalyst can be used together in the coupling reaction. Examples of the copper catalyst include copper iodide (I), copper bromide (I), copper chloride (I), and copper sulfate (II). These catalyst can be used alone or in combination of two or more kinds thereof.

The reaction can be performed in an appropriate solvent. The solvent to be used is not particularly limited as long as the solvent is inactive at the time of a reaction. Examples thereof include ethers such as diethyl ether, tetrahydrofuran, and 1,4-dioxane; hydrocarbons such as benzene, toluene, xylene, and cyclohexane; amides such as N,N-dimethylformamide and N,N-dimethylacetamide; esters such as ethyl acetate; and a mixed solvent formed of two or more of these.

Subsequently, a compound (a, b, c, d, n, R^{1a}, R³, R⁴, R⁵, and R⁶ in the formula are the same as those defined above, hereinafter, referred to as a compound (14)) represented by Formula (14) can be obtained by fusing the compound (13) and the compound (2) in the presence of a base. The condensation reaction can be performed in the same manner as in Production method 1.

A compound (15) (a, b, c, d, n, R^{1a}, R³, R⁴, R⁵, and R⁶ in the formula are the same as those defined above) can be obtained by converting structures of a tert-butoxycarbonyl (Boc) group and a nitro group in Formula (14) by a conventional method.

### [Production method 4]

Aphenylimidazole derivative (a compound represented by Formula (15b)) in which A in Formula (II) represents a carbon atom, B² represents a group represented by NR^{1c}-COR^{1a}, an imidazolyl group is an imidazole-1-yl group, and R² represents an amino group can be obtained in the same manner as Production method 3, except that a compound represented by Formula (11b) (R^{1c}, R⁶, and m in the formula are the same as those defined in Formula (II)) is used instead of the compound (11) used in Production method 3.

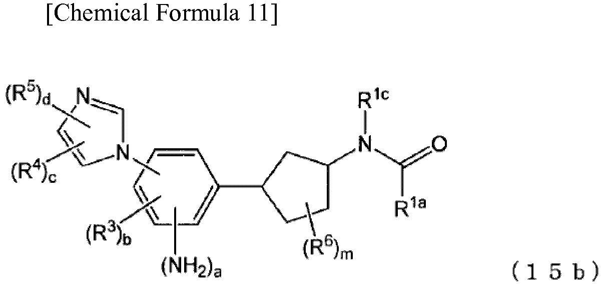

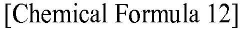

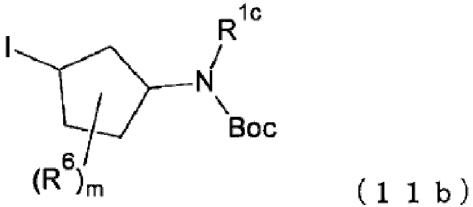

The structure of the obtained compound can be identified and verified by measuring such a spectrum using infrared spectroscopy, a nuclear magnetic resonance method, or mass spectrometry.

Since the phenylimidazole derivative according to the present invention and a salt thereof (hereinafter, also referred to as a compound of the present invention) inhibits respective enzyme activities of leukotriene A4 hydrolase, leukotriene C4 synthase, thromboxane synthase, phospholipase A2-II, and MAP kinase I, the phenylimidazole derivative and a salt thereof can be used as an active ingredient of therapeutic medicine or preventive medicine for various inflammatory diseases.

In addition, since the phenylimidazole derivative or a salt thereof inhibits activities of 15-lipoxygenase and lipid peroxidase, the phenylimidazole derivative or a salt thereof can be used as an active ingredient of therapeutic medicine or preventive medicine for various diseases caused by lipid oxidation.

Further, since the compound of the present invention exhibits effects of a treatment or prevention of retinochoroidal disorders, specifically, retinal light disorders, the compound can be used as an active ingredient of therapeutic medicine or preventive medicine for retinochoroidal disorders. More specifically, the compound of the present invention can be used for treatment or prevention of retinochroidal diseases accompanied by retinochoroidal disorders, for example, age-related macular degeneration (drusen formulation, pigment abnormality, atrophy AMD, or exudative AMD in the early stage of AMD), diabetic retinopathy (simple diabetic retinopathy, preproliferative diabetic retinopathy, or proliferative diabetic retinopathy), diabetic macular edema, proliferative vitreoretinopathy, retinopathy of prematurity, polypoidal choroidal vasculopathy, retinal hemangioma proliferation, retinal vein occulusion, retinal artery occlusion, cone dystrophy, retinal detachment, or pigmentary degeneration of the retina. Particularly, the compound of the present invention can be suitably used for treatment or prevention of atrophy AMD and a treatment or prevention of eye diseases accompanied by retinochoroidal disorders.

According to the present invention, the therapeutic medicine or preventive medicine for inflammatory diseases, diseases caused by lipid oxidation, or retinochoroidal disorders (hereinafter, also simply referred to as "therapeutic /preventive medicine) contains at least one selected from the phenylimidazole derivative represented any of Formula (I) or (II), a pharmacologically acceptable salt thereof, and metabolites thereof and preferably contains only one thereof as an active ingredient. The metabolites of the phenylimidazole derivative or a salt thereof are substances obtained as a result of a chemical reaction of the phenylimidazole derivative or a salt thereof in the body.

In the therapeutic /preventive medicine of the present invention, the therapeutically effective amount of the compound according to the present invention can be suitably selected by considering the age, the weight, the symptoms of a patient, the physical constitution of the patient, and the dosage form. The therapeutically effective daily dose is normally in the range of 0.017 mg/day to 33.3 mg/day based on 1 kg of body weight, is preferably in the range of 0.17 mg/day to 11.7 mg/day based on 1 kg of body weight, and is more preferably in the range of 1.7 mg/day to 3.3 mg/day based on 1 kg of body weight. For example, in a case where the compound of the present invention is administered to a person whose body weight is 60 kg, the dose of the compound is in the range of 1 mg/day to 2.0 g/day, is preferably in the range of 10 mg/day to 700 mg/day, and is more preferably in the range of 100 mg/day to 200 mg/day. However, the dose of the compound can be set to be in a range other than the above-described ranges by considering the symptoms of the patient, the physical constitution of the patient, the dosage form, and the like.

The therapeutic /preventive medicine of the present invention can be administered to a target using any method known to a person skilled in the art, such as an oral administration, a parenteral administration, a nasal administration, an intravascular administration, a cancer vicinity administration, a transmucosal administration, a transdermal administration, an intramuscular administration, an intranasal administration, an intravenous administration, an intradermal administration, a subcutaneous administration, a sublingual administration, an intraperitoneal administration, an intraventricular administration, an intracranial administration, an intravaginal administration, an inhalation administration, an intrarectal administration, an intratumor administration or a delivery by a ribosome. In addition, the compound can be locally administered by applying the compound to mucosal cells, for example, applying the compound to skin or an eye. Moreover, the compound can be administered to a target by an inhalation or using a method of aerosol formulation.

The therapeutic/preventive medicine of the present invention is formulated as solid or liquid formulation. Examples of the solid formulation include tablets, soft capsules, hard capsules, pills, granules, pellets, powder, and sustained-release products. Examples of the liquid formulation include solutions, suspensions, dispersants, emulsions, oils, injections, and aerosols.

The solid formulation is suitable for oral administration or nasal administration. The liquid formulation is suitable for oral administration, nasal administration, intravenous administration, intraarterial administration, or intramuscular administration. Formulations suitable for local administration to the body surface include a solution, a suspension, an emulsion, a gelling agent, an ointment, creams, lotions, and drops are exemplary examples. As suppository administration, rectal suppository or urethral suppository is an exemplary example. The therapeutic/preventive medicine of the present invention can be administered subcutaneous implantation of a sustained-release pellet. The therapeutic/preventive medicine of the present invention can be delivered to tissues by vesicles, specifically, liposomes.

The solid formulation may include a binding agent such as gum Arabic, corn starch, gelatin, carbomer, ethyl cellulose, guar gum, hydroxy propyl cellulose, hydroxy propyl methyl cellulose, povidone, or magnesium stearate; a lubricant such as stearic acid, magnesium stearate, polyethylene glycol, sodium lauryl sulfate, starch, gum Arabic, polyvinyl pyrrolidone, gelatin, or a cellulose ether derivative; an excipient such as citrate, propyl gallate, gum, starch (for example, corn starch, pregeletanized starch, or gelatinized starch), saccharide (for example, lactose, mannitol, sucrose, or dextrose), gelatin, a cellulose-based material (for example, microcrystalline cellulose), or polymethyl acrylate; a diluent such as lactose, sucrose, dicalcium phosphate, calcium carbonate, magnesium oxide, or talc; a disintegrator such as corn starch, white potato starch, alginic acid, silicon dioxide, sodium croscarmellose, crospovidone, guar gum, or sodium starch glycolate; a colorant; a flavoring agent; a fluidizing inducer; a melting agent; a buffering agent of pH and ion strength of Tris-HCl, acetate, or phosphate; an additive such as albumin or gelatin that inhibit absorption in the surface; and adjuvant.

The liquid formulation may include injectable organic ester such as propylene glycol, polyethylene glycol, or ethyl oleate; water, an alcoholic/aqueous solution, cyclodextrin, aqueous dextrose, an emulsion containing physiological saline and a buffering base, or a suspension; peanul oil, soybean oil, mineral oil, olive oil, petroleum such as sunflower seed oil or fish liver oil, animal oil, vegetable oil, and synthetic oil; a sweetening agent such as thimerosal, benzyl alcohol, or paraben; a thickener such as carbomer, colloidal silicon dioxide, ethyl cellulose, or guar gum; a melting agent, a preservative, an emulsifier such as carbomer, hydroxy propyl cellulose, or sodium lauryl sulfate; a suspending agent, a diluent, aspartame, and citric acid. In a case of parenteral administration and intravenous administration, minerals or other substances compatible with selected injection or a delivery system can be included.

Examples of components which may be contained include a cleanser such as Tween 20, Tween 80, Pluronic F68, or a bile salt; a surfactant such as a protease inhibitor or sodium lauryl sulfate; a permeation enhancer; a solubilizing agent such as cremophor, glycerol, polyethylene glycerol, benzlkonium chloride, benzyl benzoate, cyclodextrin, sorbitan ester, or stearic acid; an antioxidant such as ascorbic acid, sodium pyrosulfite, or butylated hydroxy anisole; a stabilizer such as hydroxy propyl cellulose or hydroxy propyl methyl cellulose; a fluidizing auxiliary such as colloidal silicon dioxide; a plasticizer such as diethyl phthalate or triethyl citrate; a polymer coating agent such as poloxamer or poloxamine; and a coating agent and a thin film forming agent such as ethyl cellulose, acrylate, or polymethacrylate. In addition, parenteral vehicles (for subcutaneous, intravenous, intraarterial, or intramuscular injection) may include a sodium chloride solution, Ringer's dextrose, dextrose, sodium chloride, a lactated Ringer's solution, and nonvolatile oil. Intravenous vehicles may include electrolyte supplements using a body fluid, a nutrient supplement, and Ringer's dexterous as bases.

The therapeutic/preventive medicine of the present invention may be a release controlling formulation, that is, a formulation in which the compound of the present invention is released over a certain period of time after administration. The release controlling formulation or a sustained release formulation contains a formulation in a lipophilic depot formulation (such as fatty acids, a brazing filler metal, or oil).

The therapeutic/preventive medicine of the present invention may be a rapid release formulation, that is, a formulation in which all compounds are released immediately after administration.

The therapeutic/preventive medicine of the present invention can be delivered using a release controlling system. For example, the therapeutic/preventive medicine can be administered using intravenous injection, an implantable osmotic pump, a transdermal patch, liposome, or other administration methods. The release controlling system can be placed in the vicinity of a treatment target, that is, a retinochoroidal lesion.

The therapeutic/preventive medicine of the present invention can be produced using a known method in the related art, that is, a mixing method, a granulation method or a tablet molding method. The formulation method is described in Remington's Pharmaceutical Sciences (the 18th edition, Mack Publishing Company, Easton, Pennsylvania, published in 1990).

The formulation for oral administration can be obtained by mixing the compound of the present invention with a common additive such as a vehicle, a stabilizer, or an inactive diluent according to the purpose thereof and converting the mixture into a shape suitable for administration such as a tablet, a coated tablet, a hard or soft gelatin capsule, or an aqueous, alcoholic, or oily solution according to a common method.

The formulation for parenteral formulation can be obtained by converting the compound of the present invention a solution, a suspension, or an emulsion, if necessary, together with a common substance suitable for the purpose such as a solubilizing agent or the like.

The amount of the compound according to the present invention in the therapeutic/preventive medicine of the present invention is preferably in the range of 1% by weight to 99% by weight, and more preferably in the range of 5% by weight to 75% by weight. The amount can be determined according to the administration manner. The therapeutic/preventive medicine may contain 1% by weight to 99% by weight of a pharmaceutical carrier or excipient. The remainder of the therapeutic/preventive medicine is preferably made into an appropriate pharmaceutical carrier or excipient.

The therapeutic/preventive medicine of the present invention can be administered as medicine for the above-described diseases in an arbitrary manner.

The injections may contain an aqueous solution, a non-aqueous solution, a suspension, or an emulsion which is sterile. Specific examples of an aqueous solution and a diluent of a suspension include distilled water for an injection and physiological saline. Specific examples of a non-aqueous solution and a diluent of a suspension include vegetable oil such as propylene glycol, polyethylene glycol, or olive oil; alcohols such as ethanol; and polysorbate (trade name). These compositions may further contain additives such as a tonicity agent, a preservative, a wetting agent, an emulsifier, a dispersant, a stabilizer (for example, lactose), a solubilizing agent, and a dissolution assisting agent. These can be used by being filtered through a bacteria-retaining filter, producing a solid composition of a sterilizing agent, and dissolving the composition in sterile water or a sterile solution for injection before use.

In a case where the therapeutic/preventive medicine of the present invention is used as a suppository, a carrier which is gradually dissolved in a body as a carrier, for example, a carrier in which polyoxyethylene glycol or polyethylene glycol (hereinafter, referred to as "PEG"), specifically, PEG1000 and/or PEG4000 is used and 0.5% by weight to 50% by weight of the compound of the present invention is dispersed therein, is an exemplary example.

In a case where the therapeutic/preventive medicine of the present invention is used as a liquid formulation, a solution or a suspension obtained by dissolving or dispersing 0.5% by weight to 50% by weight of the compound according to the present invention in a carrier, such as a water, a saline solution, a dextrose aqueous solution, glycerol, ethanol or the like, optionally with a pharmaceutical adjuvant. At least one from among other inhibitors for retinal oxidative disorders which are not reacted with active ingredients may be added to the compound of the present invention or other components having efficacy of medicine may be contained therein.

In a case where the compound of the present invention is used as an ophthalmic solution, at least one compound of the present invention is added to a base solvent which is normally used to obtain an aqueous solution or a suspension, and then the pH thereof can be adjusted to be preferably in a range of 4 to 10 and to be more preferably in a range of 5 to 9. It is preferable that an ophthalmic solution be subjected to a sterilization treatment in order to obtain an aseptic product and the sterilization treatment can be performed at any stage of the producing process.

The concentration of the compound of the present invention in the ophthalmic solution is preferably 0.0001% to 10% (W/V), is more preferably in the range of 0.001% to 3% (W/V), and is particularly preferably in the range of 0.01% to 1% (W/V). The dosage thereof can be once a day or divided into several times a day and several drops at each time, according to the symptoms of a patient or the body constitution of the patient. The above-described dosage is only a guide and the amount of the ophthalmic solution administered can be beyond the range.

Various additives such as a buffering agent, a tonicity agent, a preservative, a pH adjusting agent, a thickener, a chelating agent, and a solubilizing agent may be suitably added to the ophthalmic solution within the range not being reacted with the compound of the present invention.

Specific examples of the buffering agent include a citrate buffering agent, a tartrate buffering agent, an acetate buffering agent, and amino acid. Specific examples of the tonicity agent include saccharides such as sorbitol, glucose, and mannitol; polyhydric alcohols such as glycerin, polyethylene glycol, and propylene glycol; and salts such as sodium chloride. Specific examples of the preservative include paraoxybenzoates such as methyl paraoxybenzoate and ethyl paraoxybenzoate; aryl alkyl alcohols such as benzyl alcohol and phenethyl alcohol; and sorbic acid or a salt thereof. Specific examples of the pH adjusting agent include phosphoric acid and sodium hydroxide. Specific examples of the thickener include hydroxy ethyl cellulose, hydroxy propyl cellulose, methyl cellulose, hydroxy propyl methyl cellulose, carboxy methyl cellulose, and salts of these. Specific examples of the chelating agent include sodium edetate, sodium citrate, and fused sodium phosphate. Specific examples of the solubilizing agent include ethanol and polyoxyethylene hardened castor oil.

In a case where the compound of the present invention is an eye ointment, at least one compound from among the compounds of the present invention can be mixed with an eye ointment which is normally used, for example, purified lanolin, white petrolatum, macrogol, plastibase, or liquid paraffin and it is preferable that the mixture be subjected to a sterilization treatment in order to obtain an aseptic product.

The concentration of the compound of the present invention in the eye ointment is preferably 0.0001% to 10% (W/W), is more preferably in the range of 0.001% to 3% (W/W), and is particularly preferably in the range of 0.01% to 1% (W/W). The dosage thereof can be once a day or divided into several times a day according to the symptoms of a patient or the body constitution of the patient and several drops can be respectively administered. The above-described dosage is only a guide and the eye ointment can be administered beyond that range.

### Examples

The present invention will be described in detail with reference to Examples below. However, the technical scope of the present invention is not limited to the following Examples.

### (Example 1)

### Production of 4-acetyl-1-(4-amino-3-imidazole-1-yl-phenyl)piperazine (compound 1-1)

### Process 1: production of 2-imidazole-1-yl-4-fluoronitrobenzene

4.00 g of 2,4-difluoronitrobenzene, 1.71 g of imidazole, and 3.82 g of potassium carbonate were suspended in 40 mL of N-N-dimethylformamide and the solution was stirred at room temperature for one day. Water was added to the reaction solution, the solution was extracted by chloroform three times, washed with saturated saline, dried by anhydrous magnesium sulfate, and concentrated under reduced pressure. The residue was purified with silica gel column chromatography (chloroform:ethyl acetate =1:1 (volume ratio)), thereby obtaining 3.87 g of a target object.

### Process 2: production of 2-imidazole-1-yl-4-(4-acetylpiperazine-1-yl) nitrobenzene

3.87 g of 2-imidazole-1-yl-4-fluoronitrobenzene, 2.64 g of 1-acetylpiperazine, and 3.36 g of potassium carbonate were suspended in 9 mL of dimethyl sulfoxide, and the solution was stirred at 100°C for 2 hours. Water was added to the reaction solution, and deposited crystals were separated by filtration, followed by washing with water and ether in order. Chloroform and methanol were added to the obtained solid, followed by drying by anhydrous magnesium sulfate and concentrating under reduced pressure. The residue was purified with silica gel column chromatography (chloroform:methanol =9:1 (volume ratio)), thereby obtaining 5.78 g of a target object.

### Process 3: production of 4-acetyl-1-(4-amino-3-imidazole-1-yl-phenyl)piperazine

3 g of 2-imidazole-1-yl-4-(4-acetylpiperazine-1-yl)nitrobenzene and 0.2 g of 10% Pd/C were added to 50 mL of ethanol and the solution was stirred at 50°C and at a hydrogen pressure of 0.45 MPa for 1.5 hours. After the solution was cooled, the reaction solution was filtered using celite, the solvent was distilled under reduced pressure, and the residue was purified with silica gel column chromatography (Fuji Silysia Silica Gel (NH): chloroform). The obtained crystals were recrystallized using ethyl acetate-methanol-hexane, thereby obtaining 1.6 g of a target object having a melting point of 94°C to 97°C.

### (Example 2)

### Process 1: production of 3-acetylamino-1-(4-nitro-3-imidazole-1-yl-phenyl)pyrrolidine

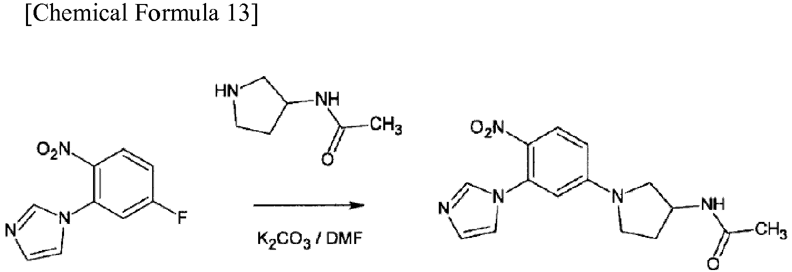

2.07 g of 2-imidazole-1-yl-4-fluoronitrobenzene, 1.54 g of 3-acetylaminopyrrolidine, and 1.66 g of potassium carbonate were suspended in 20 mL of N,N-dimethylformamide, and the solution was stirred at 80°C for 2 hours. The reaction solution was dropwise added to ice-water, and deposited crystals were separated by filtration and washed with water, thereby obtaining 2.5 g of a target object.

### Process 2: production of 3-acetylamino-1-(4-amino-3-imidazole-1-yl-phenyl)pyrrolidine

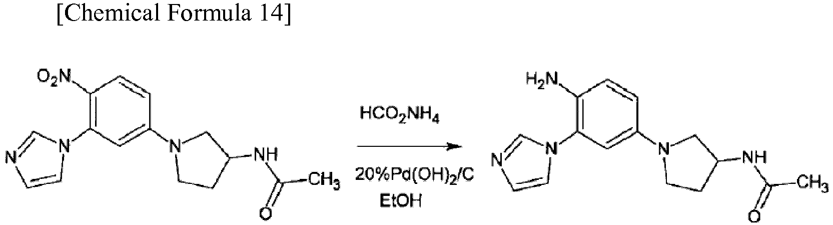

2 g of 3-acetylamino-1-(4-nitro 3-imidazole-1-yl-phenyl)pyrrolidine, 1.2 g of 20% Pd(OH)₂/C, and 1.5 g of ammonium formate were added to 25 mL of ethanol and the solution was refluxed for 30 minutes. After the solution was cooled, the reaction solution was filtered using celite, the solvent was distilled under reduced pressure, and the residue was purified with silica gel column chromatography (Fuji Silysia Silica Gel (NH): chloroform). The obtained crystals were recrystallized using ethyl acetate-methanol-hexane, thereby obtaining 1.02 g of a target object having a melting point of 169°C to 171°C.

Examples of the compound of the present invention including compounds described in Examples above are listed in Tables 1 to 4. In addition, Table 1 shows a compound represented by Formula (Ia), Table 2 shows a compound represented by Formula (Ib), Table 3 shows a compound represented by Formula (IIa), and Table 4 shows a compound represented by Formula (IIb). Me represents a methyl group, Et represents an ethyl group, ⁿPr represents a n-propyl group, ⁱPr represents an isopropyl group, ^{c}Pr represents a cyclopropyl group, ⁿBu represents a n-butyl group, ⁱBu represents an isobutyl group, and Ac represents an acetyl group. A, B¹, B², R^{1a}, R^{1c}, (R²)ₐ, (R³)_{b}, (R⁴)_{c}, (R⁵)_{d}, (R⁶)ₙ, and (R⁶)ₘ represent a substituent in a compound represented by Formula (Ia), (Ib), (IIa), or (IIb). Further, *1 represents a binding position of an imidazole-1-yl group.

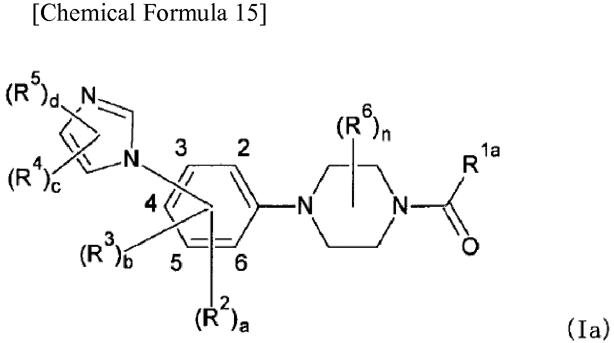

### [Table 1]

**Table 1**

| No. | R^{1a} | *1 | (R²)ₐ | (R³)_{b} | (R⁴)_{c} | (R⁵)_{d} | (R⁶)ₙ |
|---|---|---|---|---|---|---|---|
| 1-1 | Me | 3-position | 4-NH₂ | - | - | - | - |
| 1-2 | Me | 3-position | 4-NH₂ | - | - | 2-Me | - |
| 1-3 | Me | 3-position | 4-NH₂ | - | - | 4-Me | - |
| 1-4 | Me | 3-position | 4-NH₂ | - | - | 2-CF₃ | - |
| 1-5 | Me | 3-position | 4-NHAc | - | - | - | - |
| 1-6 | Me | 3-position | 4-NHCOAc | - | - | - | - |
| 1-7 | Me | 3-position | 4-NMe₂ | - | - | - | - |
| 1-8 | H | 3-position | 4-NH₂ | 2-F | - | - | - |
| 1-9 | ^{c}Pr | 3-position | 4-NH₂ | - | - | - | - |
| 1-10 | O^{t}Bu | 3-position | 4-NH₂ | - | - | - | - |
| 1-11 | CH₂OMe | 3-position | 4-NH₂ | - | - | - | - |
| 1-12 | CH₂CF₃ | 3-position | 4-NH₂ | 2-Me | - | - | - |
| 1-13 | CH=CH₂ | 3-position | 4-NH₂ | - | 4-CN | - | - |
| 1-14 | NMe₂ | 3-position | 4-NH₂ | - | - | - | - |
| 1-15 | Morpholin-4-yl | 3-position | 4-NH₂ | - | - | - | - |
| 1-16 | Me | 3-position | 4-OMe | - | - | - | - |
| 1-17 | Me | 3-position | 4-OH | - | - | - | - |
| 1-18 | Me | 3-position | 4-OAc | - | - | - | - |
| 1-19 | Et | 3-position | 4-CN | - | - | - | - |
| 1-20 | Me | 3-position | 4-CH₂NHⁱPr | - | 4-CH₂OH | - | - |
| 1-21 | Me | 3-position | 4-NHSO₂Me | - | | 2-Me | - |
| 1-22 | Me | 3-position | 4-N=CH(NMe₂) | - | - | 4-Me | - |
| 1-23 | Me | 3-position | 4-NHCOCF₃ | - | - | 5-Me | - |
| 1-24 | Me | 3-position | 4-NHCHO | - | - | 2,4-Me₂ | - |
| 1-25 | Me | 3-position | 4-NHCOEt | - | - | 2-ⁱPr | - |
| 1-26 | Me | 3-position | 4-NHCO^{c}Pr | - | - | 4-CF₃ | - |
| 1-27 | Me | 3-position | 4-NHCO^{c}Pr | - | - | 2-Me | - |
| 1-28 | Me | 3-position | 4-NHCOCH₂OMe | - | - | - | - |
| 1-29 | Me | 3-position | 4-NHCOCH₂NMe₂ | - | - | - | - |
| 1-30 | Me | 3-position | 4-NHEt | - | - | - | - |
| 1-31 | Me | 3-position | 4-NHⁱBu | - | - | - | - |
| 1-32 | Me | 3-position | 4-NHCH₂^{c}Pr | - | - | - | - |

### [Table 2]

Continuation of Table 1

| No. | R^{1a} | *1 | (R²)ₐ | (R³)_{b} | (R⁴)_{c} | (R⁵)_{d} | (R⁶)ₙ |
|---|---|---|---|---|---|---|---|
| 1-33 | Me | 3-position | 4-NHCH₂C=CH | - | - | - | - |
| 1-34 | Me | 3-position | 4-NHCO₂Me | - | - | - | - |
| 1-35 | Me | 3-position | 2-NH₂ | - | - | 2-Me | - |
| 1-36 | Me | 3-position | 2-NH₂ | - | - | 3-Me | - |
| 1-37 | Me | 3-position | 4-NH₂-5-NHⁱPr | - | - | - | - |
| 1-38 | Me | 3-position | 4-NH₂-5-NMe₂ | - | - | - | - |
| 1-39 | Me | 3-position | 4-NH₂-5-OMe | - | - | - | - |
| 1-40 | Me | 3-position | 4-NH₂-5-OEt | - | - | - | - |
| 1-41 | Me | 4-position | 3-NH₂ | - | - | - | - |
| 1-42 | Me | 4-position | 3-NHAc | - | - | - | - |
| 1-43 | Me | 4-position | 3-NHCOAc | - | - | - | - |
| 1-44 | Me | 4-position | 3-NMe₂ | - | - | - | - |
| 1-45 | H | 4-position | 3-NH₂ | - | - | - | - |
| 1-46 | ^{c}Pr | 4-position | 3-NH₂ | - | - | - | - |
| 1-47 | O^{t}Bu | 4-position | 3-NH₂ | - | - | - | - |
| 1-48 | CH₂OMe | 4-position | 3-NH₂ | - | - | - | - |
| 1-49 | CH₂CF₃ | 4-position | 3-NH₂ | - | - | - | - |
| 1-50 | NMe₂ | 4-position | 3-NH₂ | - | - | - | - |
| 1-51 | Morpholin-4-yl | 4-position | 3-NH₂ | - | - | - | - |
| 1-52 | Me | 4-position | 3-OMe | - | - | - | - |
| 1-53 | Me | 2-position | 4-NH₂ | - | - | - | - |
| 1-54 | Me | 2-position | 4-NHAc | - | - | - | - |
| 1-55 | Me | 2-position | 4-NHCOAc | - | - | - | - |
| 1-56 | Me | 2-position | 4-NMe₂ | - | - | - | - |
| 1-57 | H | 2-position | 4-NH₂ | - | - | - | - |
| 1-58 | ^{c}Pr | 2-position | 4-NH₂ | - | - | - | - |
| 1-59 | O^{t}Bu | 2-position | 4-NH₂ | - | - | - | - |
| 1-60 | CH₂OMe | 2-position | 4-NH₂ | - | - | - | - |
| 1-61 | CH₂CF₃ | 2-position | 4-NH₂ | - | - | - | - |
| 1-62 | NMe₂ | 2-position | 4-NH₂ | - | - | - | - |
| 1-63 | Morpholin-4-yl | 2-position | 4-NH₂ | - | - | - | - |
| 1-64 | Me | 2-position | 4-OMe | - | - | - | - |

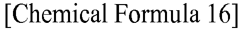

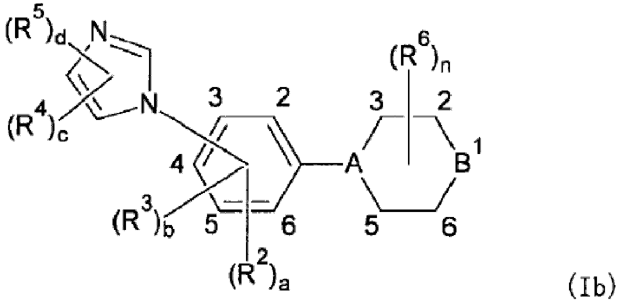

### [Table 3]

**Table 2**

| No. | A | B¹ | *1 | (R²)ₐ | (R³)_{b} | (R⁴)_{c} | (R⁵)_{d} | (R⁶)ₙ |
|---|---|---|---|---|---|---|---|---|
| 2-1 | N | N-C(Me)=NOH | 3-position | 4-NH₂ | - | - | - | - |
| 2-2 | N | N-C(Me)=NOH | 3-position | 4-NHAc | - | - | - | - |
| 2-3 | N | N-C(Me)=NOH | 3-position | 4-NHCOAc | - | - | - | - |
| 2-4 | N | N-C(Me)=NOH | 3-position | 4-NMe₂ | - | - | - | - |
| 2-5 | N | N-SO₂Me | 3-position | 4-NH₂ | - | - | - | - |
| 2-6 | N | N-SO₂Me | 3-position | 4-NH₂ | - | - | - | - |
| 2-7 | N | N-SO₂Me | 3-position | 4-NHAc | - | - | - | - |
| 2-8 | N | N-SO₂Me | 3-position | 4-NHCOAc | - | - | - | - |
| 2-9 | N | N-SO₂Me | 3-position | 4-NMe₂ | - | - | - | - |
| 2-10 | N | N-SO₂^{c}Pr | 3-position | 4-NH₂ | - | - | - | - |
| 2-11 | N | N-SO₂CH₂OMe | 3-position | 4-NH₂ | - | - | - | - |
| 2-12 | N | N-SO₂CH₂CF₃ | 3-position | 4-NH₂ | - | - | - | - |
| 2-13 | N | N-SO₂NMe₂ | 3-position | 4-NH₂ | - | - | - | - |
| 2-14 | CH | N-CO₂^{t}Bu | 3-position | 4-NH₂ | - | - | - | - |
| 2-15 | CH | N-COMe | 3-position | 4-NH₂ | - | - | - | - |
| 2-16 | CH | N-COMe | 3-position | 4-NHAc | - | - | - | - |
| 2-17 | CH | N-COMe | 3-position | 4-NHCOAc | - | - | - | - |
| 2-18 | CH | N-COMe | 3-position | 4-NMe₂ | - | - | - | - |
| 2-19 | CH | N-CO^{c}Pr | 3-position | 4-NH₂ | - | - | - | - |
| 2-20 | CH | N-COCH₂OMe | 3-position | 4-NH₂ | - | - | - | - |
| 2-21 | CH | N-COCH₂CF₃ | 3-position | 4-NH₂ | - | - | - | - |
| 2-22 | CH | N-CONMe₂ | 3-position | 4-NH₂ | - | - | - | - |
| 2-23 | CH | N-COMe | 3-position | 4-NHSO₂Me | - | - | - | - |
| 2-24 | CH | N-COMe | 3-position | 4-N=CH(NMe₂) | - | - | - | - |
| 2-25 | CH | N-COMe | 3-position | 4-NHCOCF₃ | - | - | - | - |

### [Table 4]

**Continuation of Table 2**

| No. | A | B¹ | *1 | (R²)ₐ | (R³)_{b} | (R⁴)_{c} | (R⁵)_{d} | (R⁶)ₙ |
|---|---|---|---|---|---|---|---|---|
| 2-26 | CH | N-COMe | 3-position | 4-NHCHO | - | - | - | - |
| 2-27 | CH | N-COMe | 3-position | 4-NHCOEt | - | - | - | - |
| 2-28 | CH | N-COMe | 3-position | 4-NHCO^{c}Pr | - | - | - | - |
| 2-29 | CH | N-COMe | 3-position | 4-NHCO^{c}Pr | - | - | - | - |
| 2-30 | CH | N-COMe | 3-position | 4-NHCOCH₂OMe | - | - | - | - |
| 2-31 | CH | N-COMe | 3-position | 4-NHCOCH₂NMe₂ | - | - | - | - |
| 2-32 | CH | N-COMe | 3-position | 4-NHEt | - | - | - | - |
| 2-33 | CH | N-COMe | 3-position | 4-NHⁱBu | - | - | - | - |
| 2-34 | CH | N-COMe | 3-position | 4-NHCH₂ⁱPr | - | - | - | - |
| 2-35 | CH | N-COMe | 3-position | 4-NHCH₂C≡CH | - | - | - | - |
| 2-36 | CH | N-COMe | 3-position | 4-NHCO₂Me | - | - | - | - |
| 2-37 | N | C=O | 3-position | 4-NH₂ | - | - | - | 3-(CH₂)₃-5 |
| 2-38 | N | C=O | 3-position | 4-NH₂ | - | - | - | 3-(CH₂)₂-5 |
| 2-39 | N | C=O | 3-position | 4-NHAc | - | - | - | 3-(CH₂)₃-5 |
| 2-40 | N | C=O | 3-position | 4-NHCOAc | - | - | - | 3-(CH₂)₃-5 |
| 2-41 | N | C=O | 3-position | 4-NMe₂ | - | - | - | 3-(CH₂)₃-5 |

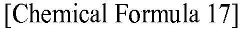

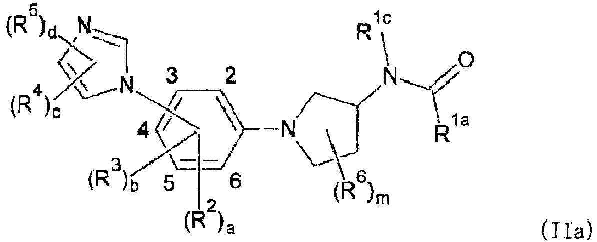

### [Table 5]

**Table 3**

| No. | R^{1a} | R^{1c} | *1 | (R²)ₐ | (R³)_{b} | (R⁴)_{c} | (R⁵)_{d} | (R⁶)ₘ |
|---|---|---|---|---|---|---|---|---|
| 3-1 | Me | H | 3-position | 4-NH₂ | - | - | - | - |
| 3-2 | Me | H | 3-position | 4-NH₂ | - | - | 2-Me | - |
| 3-3 | Me | H | 3-position | 4-NH₂ | - | - | 4-Me | - |
| 3-4 | Me | H | 3-position | 4-NH₂ | - | - | 2-CF₃ | - |
| 3-5 | Me | H | 3-position | 4-NHAc | - | - | - | - |
| 3-6 | Me | H | 3-position | 4-NHCOAc | - | - | - | - |
| 3-7 | Me | H | 3-position | 4-NMe₂ | - | - | - | - |
| 3-8 | H | H | 3-position | 4-NH₂ | 2-F | - | - | - |
| 3-9 | ^{c}Pr | H | 3-position | 4-NH₂ | - | - | - | - |
| 3-10 | O^{t}Bu | H | 3-position | 4-NH₂ | - | - | - | - |
| 3-11 | CH₂OMe | H | 3-position | 4-NH₂ | - | - | - | - |
| 3-12 | CH₂CF₃ | H | 3-position | 4-NH₂ | 2-Me | - | - | - |
| 3-13 | CH=CH₂ | H | 3-position | 4-NH₂ | - | 4-CN | - | - |
| 3-14 | NMe₂ | H | 3-position | 4-NH₂ | - | - | - | - |
| 3-15 | Morpholin-4-yl | H | 3-position | 4-NH₂ | - | - | - | - |
| 3-16 | Me | H | 3-position | 4-OMe | - | - | - | - |
| 3-17 | Me | H | 3-position | 4-OH | - | - | - | - |
| 3-18 | Me | H | 3-position | 4-OAc | - | - | - | - |
| 3-19 | Et | H | 3-position | 4-CN | - | - | - | - |
| 3-20 | Me | H | 3-position | 4-CH₂NHⁱPr | - | 4-CH₂OH | - | - |
| 3-21 | Me | H | 3-position | 4-NHSO₂Me | - | - | 2-Me | - |
| 3-22 | Me | H | 3-position | 4-N=CH(NMe₂) | - | - | 4-Me | - |
| 3-23 | Me | H | 3-position | 4-NHCOCF₃ | - | - | 5-Me | - |
| 3-24 | Me | H | 3-position | 4-NHCHO | - | - | 2,4-Me₂ | - |
| 3-25 | Me | H | 3-position | 4-NHCOEt | - | - | 2-ⁱPr | - |
| 3-26 | Me | H | 3-position | 4-NHCO^{c}Pr | - | - | 4-CF₃ | - |
| 3-27 | Me | H | 3-position | 4-NHCOⁱPr | - | - | 2-Me | - |
| 3-28 | Me | H | 3-position | 4-NHCOCH₂OMe | - | - | - | - |
| 3-29 | Me | H | 3-position | 4-NHCOCH₂NMe₂ | - | - | - | - |
| 3-30 | Me | H | 3-position | 4-NHEt | - | - | - | - |
| 3-31 | Me | H | 3-position | 4-NHⁱBu | - | - | - | - |
| 3-32 | Me | H | 3-position | 4-NHCH₂^{c}Pr | - | - | - | - |

### [Table 6]

**Continuation of Table 3**

| No. | R^{1a} | R^{1c} | *1 | (R²)ₐ | (R³)_{b} | (R⁴)_{c} | (R⁵)_{d} | (R⁶)ₘ |
|---|---|---|---|---|---|---|---|---|
| 3-33 | Me | H | 3-position | 4-NHCH₂C≡CH | - | - | - | - |
| 3-34 | Me | H | 3-position | 4-NHCO₂Me | - | - | - | - |
| 3-35 | Me | H | 3-position | 2-NH₂ | - | - | 2-Me | - |
| 3-36 | Me | H | 3-position | 2-NH₂ | - | - | 3-Me | - |
| 3-37 | Me | H | 3-position | 4-NH₂-5-NHⁱPr | - | - | - | - |
| 3-38 | Me | H | 3-position | 4-NH₂-5-NMe₂ | - | - | - | - |
| 3-39 | Me | H | 3-position | 4-NH₂-5-OMe | - | - | - | - |
| 3-40 | Me | H | 3-position | 4-NH₂-5-OEt | - | - | - | - |
| 3-41 | Me | H | 4-position | 3-NH₂ | - | - | - | - |
| 3-42 | Me | H | 4-position | 3-NHAc | - | - | - | - |
| 3-43 | Me | H | 4-position | 3-NHCOAc | - | - | - | - |
| 3-44 | Me | H | 4-position | 3-NMe₂ | - | - | - | - |
| 3-45 | H | H | 4-position | 3-NH₂ | - | - | - | - |
| 3-46 | ^{c}Pr | H | 4-position | 3-NH₂ | - | - | - | - |
| 3-47 | O^{t}Bu | H | 4-position | 3-NH₂ | - | - | - | - |
| 3-48 | CH₂OMe | H | 4-position | 3-NH₂ | - | - | - | - |
| 3-49 | CH₂CF₃ | H | 4-position | 3-NH₂ | - | - | - | - |
| 3-50 | NMe₂ | H | 4-position | 3-NH₂ | - | - | - | - |
| 3-51 | Morpholin-4-yl | H | 4-position | 3-NH₂ | - | - | - | - |
| 3-52 | Me | H | 4-position | 3-OMe | - | - | - | - |
| 3-53 | Me | H | 2-position | 4-NH₂ | - | - | - | - |
| 3-54 | Me | H | 2-position | 4-NHAc | - | - | - | - |
| 3-55 | Me | H | 2-position | 4-NHCOAc | - | - | - | - |
| 3-56 | Me | H | 2-position | 4-NMe₂ | - | - | - | - |
| 3-57 | H | H | 2-position | 4-NH₂ | - | - | - | - |
| 3-58 | ^{c}Pr | H | 2-position | 4-NH₂ | - | - | - | - |
| 3-59 | O^{t}Bu | H | 2-position | 4-NH₂ | - | - | - | - |
| 3-60 | CH₂OMe | H | 2-position | 4-NH₂ | - | - | - | - |
| 3-61 | CH₂CF₃ | H | 2-position | 4-NH₂ | - | - | - | - |
| 3-62 | NMe₂ | H | 2-position | 4-NH₂ | - | - | - | - |
| 3-63 | Morpholin-4-yl | H | 2-position | 4-NH₂ | - | - | - | - |
| 3-64 | Me | H | 2-position | 4-OMe | - | - | - | - |

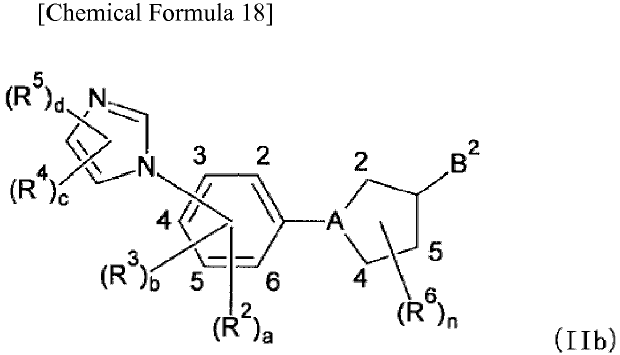

### [Table 7]

**Table 4**

| No. | A | B² | *1 | (R²)ₐ | (R³)_{b} | (R⁴)_{c} | (R⁵)_{d} | (R⁶)ₘ |
|---|---|---|---|---|---|---|---|---|
| 4-1 | N | NH-C(Me)=NOH | 3-position | 4-NH₂ | - | - | - | - |
| 4-2 | N | NH-C(Me)=NOH | 3-position | 4-NHAc | - | - | - | - |
| 4-3 | N | NH-C(Me)=NOH | 3-position | 4-NHCOAc | - | - | - | - |
| 4-4 | N | NH-C(Me)=NOH | 3-position | 4-NMe₂ | - | - | - | - |
| 4-5 | N | NH-SO₂Me | 3-position | 4-NH₂ | - | - | - | - |
| 4-6 | N | NH-SO₂Me | 3-position | 4-NH₂ | - | - | - | - |
| 4-7 | N | NH-SO₂Me | 3-position | 4-NHAc | - | - | - | - |
| 4-8 | N | NH-SO₂Me | 3-position | 4-NHCOAc | - | - | - | - |
| 4-9 | N | NH-SO₂Me | 3-position | 4-NMe₂ | - | - | - | - |
| 4-10 | N | N-SO₂^{c}Pr | 3-position | 4-NH₂ | - | - | - | - |
| 4-11 | N | NH-SO₂CH₂OMe | 3-position | 4-NH₂ | - | - | - | - |
| 4-12 | N | NH-SO₂CH₂CF₃ | 3-position | 4-NH₂ | - | - | - | - |
| 4-13 | N | NH-SO₂NMe₂ | 3-position | 4-NH₂ | - | - | - | - |
| 4-14 | CH | NH-CO₂^{t}Bu | 3-position | 4-NH₂ | - | - | - | - |
| 4-15 | CH | NH-COMe | 3-position | 4-NH₂ | - | - | - | - |
| 4-16 | CH | NH-COMe | 3-position | 4-NHAc | - | - | - | - |
| 4-17 | CH | NH-COMe | 3-position | 4-NHCOAc | - | - | - | - |
| 4-18 | CH | NH-COMe | 3-position | 4-NMe₂ | - | - | - | - |
| 4-19 | CH | NH-CO^{c}Pr | 3-position | 4-NH₂ | - | - | - | - |
| 4-20 | CH | NH-COCH₂OMe | 3-position | 4-NH₂ | - | - | - | - |
| 4-21 | CH | NH-COCH₂CF₃ | 3-position | 4-NH₂ | - | - | - | - |
| 4-22 | CH | NH-CONMe₂ | 3-position | 4-NH₂ | - | - | - | - |
| 4-23 | CH | NH-COMe | 3-position | 4-NHSO₂Me | - | - | - | - |
| 4-24 | CH | NH-COMe | 3-position | 4-N=CH(NMe₂) | - | - | - | - |
| 4-25 | CH | NH-COMe | 3-position | 4-NHCOCF₃ | - | - | - | - |

### [Table 8]

**Continuation of Table 4**

| No. | A | B² | *1 | (R²)ₐ | (R³)_{b} | (R⁴)_{c} | (R⁵)_{d} | (R⁶)ₘ |
|---|---|---|---|---|---|---|---|---|
| 4-26 | CH | NH-COMe | 3-position | 4-NHCHO | - | - | - | - |
| 4-27 | CH | NH-COMe | 3-position | 4-NHCOEt | - | - | - | - |
| 4-28 | CH | NH-COMe | 3-position | 4-NHCO^{c}Pr | - | - | - | - |
| 4-29 | CH | NH-COMe | 3-position | 4-NHCOⁱPr | - | - | - | - |
| 4-30 | CH | NH-COMe | 3-position | 4-NHCOCH₂OMe | - | - | - | - |
| 4-31 | CH | NH-COMe | 3-position | 4-NHCOCH₂NMe₂ | - | - | - | - |
| 4-32 | CH | NH-COMe | 3-position | 4-NHEt | - | - | - | - |
| 4-33 | CH | NH-COMe | 3-position | 4-NHⁱBu | - | - | - | - |
| 4-34 | CH | NH-COMe | 3-position | 4-NHCH₂^{c}Pr | - | - | - | - |
| 4-35 | CH | NH-COMe | 3-position | 4-NHCH₂C≡CH | - | - | - | - |
| 4-36 | CH | NH-COMe | 3-position | 4-NHCO₂Me | - | - | - | - |

Physical properties of some compounds listed in Tables 1 to 4 will be shown below. mp indicates a melting point and vis. indicates that the compound is a viscous oil-like compound.
1-1: mp 94°C to 97°C
1-2: mp 78°C to 83°C
1-5: mp 183°C to 185°C
1-6: mp 147°C to 149°C
1-7: mp 152°C to 154°C
1-8: mp 183°C to 184°C
1-9: vis.
1-10: vis.
1-11: vis.
1-12: mp 217°C to 219°C
1-14: mp 149°C to 150°C
1-15: vis.
1-16: vis.
2-4: mp 185°C to 188°C
2-5: mp 178°C to 179°C
2-14: vis.
2-37: vis.
3-1: mp 169°C to 171°C

### (Preparation of formulation)

A formulation containing the compound of the present invention was prepared by the following method.

Formulation Example 1: Preparation of an oral agent (active ingredient 10 mg tablets)

Compound of the present invention: 10.0 mg
Lactose: 81.4 mg
Cone starch: 20.0 mg
Hydroxy propyl cellulose: 4.0 mg
Carboxy methyl cellulose calcium: 4.0 mg
Magnesium stearate: 0.6 mg
Total: 120.0 mg

50 g of the compound of the present invention, 407 g of lactose, and 100 g of cone starch were uniformly mixed with each other using a fluidized granulation coating device (manufactured by OKAWARAMFG CO., LTD.) so as to obtain the composition described above. 200 g of a 10% hydroxy propyl cellulose aqueous solution was sprayed to the mixture and granulated. After the mixture was dried, the mixture passed through a 20 mesh sieve, 20 g of carboxy methyl cellulose calcium and 3 g of magnesium stearate were added thereto, and then tablets, each of which has a weight of 120 mg, were obtained using a stamp mill having a dimension of 7 mm x 8.4 R with a rotary tableting machine (manufactured by HATA TEKKOSHO CO., LTD.).

### (Retinal light disorder model test)

The usefulness (improvement rate with respect to retinal light disorders) of the compound of the invention was evaluated using a mouse model of retinal light disorders, generally used as a model of retinal light disorders, (Investigative Ophthalmology & Visual Science, 2002; vol. 43; pp. 1162 to 1167). Here, the improvement rate with respect to the retinal light disorders was calculated based on the thickness of an outer nuclear layer (hereinafter, referred to as "ONL"), which is strongly interfered especially by light application among a retinal cell layers , in a tissue specimen of a perpendicular surface of an eyeball.

Moreover, in the present test, the ONL of a light application group to which only a base was administered was degraded to 30% to 50% of a normal light group.

### 1. Preparation of test compound administration liquid

A test compound was dissolved or suspended in a solvent (1% (w/v) methyl cellulose aqueous solution) and then an administration liquid was prepared so that the test compound to be 30 mg/kg body weight. The dose was set to 10 mL/kg body weight.

### 2. Test and measurement method

1) Test compound group:
   A 5-week-old BALB/cCr-based male mouse (weight of approximately 20 g) was bred in a dark place for 48 hours. Subsequently, the test compound administration liquid was orally administered (30 mg/kg). After 30 minutes, 6000 lux to 5000 lux of white light was continuously applied to the mouse for 2 hours. Next, the mouse was bred in a breeding room with 50 lux of light for 4 days.
   An anesthetic (secobarbital sodium) was intraperitoneally administered to anesthetize the mouse and then the mouse was exsanguinated to die. The top portion of the right eyeball was marked with a thread and the eyeball was extracted. The extracted eyeball was immersed in a fixing solution I (4% formaldehyde + 0.25% glutaraldehyde) for approximately 3 hours. Next, the cornea was removed and then further immersed in the fixing solution for 24 hours. Subsequently, the eyeball was immersed in a fixing solution II (10% neutral buffered formalin) and then fixed. The eyeball was cut along with a longitudinal section including the optic nerve papilla and subjected to hematoxylin and eosin staining, thereby obtaining a tissue specimen.
2) Light application group:
   The operation was performed in the same manner as the test compound group, except that only the solvent was orally administered instead of the test compound administration liquid.
3) Normal light group:
   The operation was performed in the same manner as the test compound group, except that only the solvent was orally administered instead of the test compound administration liquid and the mouse was not continuously irradiated with 6000 lux to 5000 lux of white light for 2 hours.

### 3. Evaluation method of tissue specimen

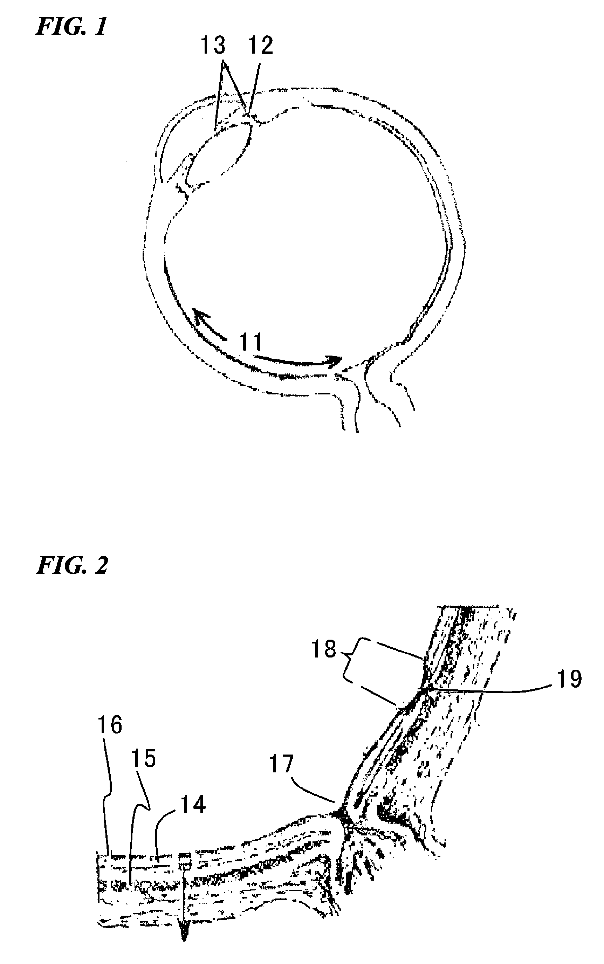

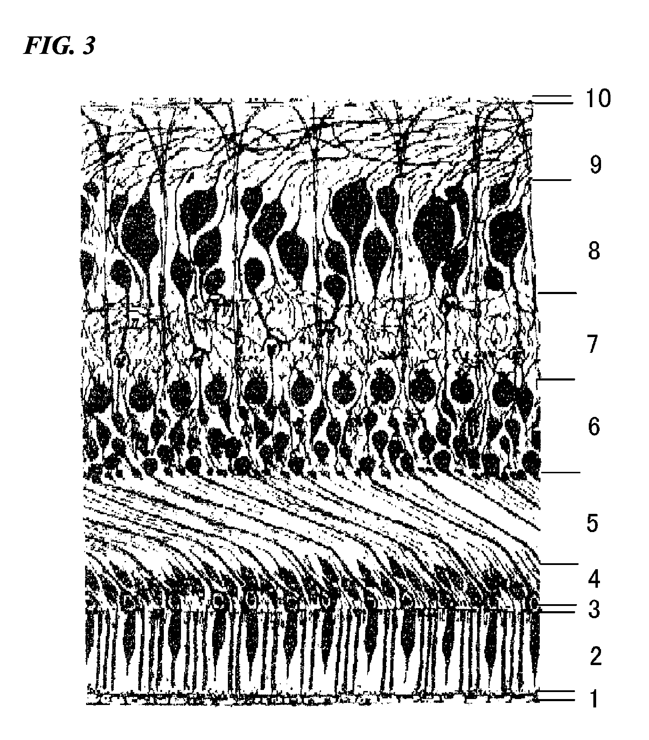

A retina 14 is formed of ten layers of a retinal pigment epithelial layer 1, a visual cell layer 2, an external limiting membrane 3, an ONL 4, an outer plexiform layer 5, an inner nuclear layer 6, an inner plexiform layer 7, a ganglion cell layer 8, an optic nerve fiber layer 9, and an internal limiting membrane 10, from outside to inside (see Figs. 1 to 3). Degeneration is started due to light application from visual cells, outer segments and inner segments of the visual cells are lost, and the ONL is reduced (Opthalmology clinic practice; 2002; vol. 5; pp. 93).

The ONL thickness in the tissue specimen was measured for every 200 µm between the optic nerve papilla to where upwardly spaced by 1 mm from the optic nerve papilla using Lumina Vision. The area (AUC) from the optic nerve papilla to where upwardly spaced by 1 mm from the optic papilla in the graph was calculated as an ONL area.

### 4. Calculation formula of ONL thickness improvement rate (%)

ONL thickness improvement rate (%) = (1 - (AUC of normal light group - AUC of test compound group)/(AUC of normal light group - AUC of light application group) x 100

### 5. Statistical processing

FT test between the normal light group and the light application group (one-side test) and between the light application group and the test compound group (two-side test) was performed according to the ONL area.

### 6. Test results

As a part of the test results, the ONL thickness improvement rates of the case where the compounds of the present invention 1-1, 1-2, 1-8, 1-9, 1-10, 1-11, 1-12, 1-14, 1-15, 2-5, 2-14, 2-37, and 3-1 were used are shown in Table 5.

In addition, when 2-imidazole-1-yl-4-(acetylpiperazine-1-yl)nitrobenzene described in PTL 2 was used as a reference example, the ONL thickness improvement rate was 29.2%.

### [Table 9]

**Table 5**

| No. | ONL thickness improvement rate (%) |
|---|---|
| 1-1 | 100.6 |
| 1-2 | 64.8 |
| 1-8 | 106.3 |
| 1-9 | 83.1 |
| 1-10 | 31.5 |
| 1-11 | 104 |
| 1-12 | 52.3 |
| 1-14 | 42.2 |
| 1-15 | 45.7 |
| 2-5 | 93.1 |
| 2-14 | 55.6 |
| 2-37 | 94.1 |
| 3-1 | 87.2 |

As seen from the results of the FT examination, the compound of the present invention significantly suppressed a decrease of ONL thickness in the animal models of the retinal light disorders when administered orally at 30 mg/kg. In other words, the compound of the present invention exhibited treatment and/or prevention effects for retinal light disorders in the models of the retinal light disorders.

### (Cytochrome P450 inhibitory action)

An inhibitory action of the compound of the present invention with respect to CYP2D6 (dextromethorphan was used as a substrate) and CYP3A4 (midazolam and testosterone were used as substrates) which are isozymes of cytochrome P450 (hereinafter, referred to as "CYP") serving as a major drug-metabolizing enzyme was examined using human liver microsomes.

Using human liver microsomes pooled as an enzyme source, 1.3 mM NADP as a coenzyme, 3.3 mM D-glucose-6-phosphate, and 0.4 U/mL D-glucose-6-phosphate dehydrogenase, the corresponding substrate (5 µM dextromethorphan for CYP2D6, 5 µM midazolam or 50 µM testosterone for CYP3A4) and the compound of the present invention were added such that the final concentration became 0.1, 1, 10 µM and then incubated at 37°C for 10 minutes. The inhibition rate (%) of the compound of the present invention in each concentration was calculated by quantifying metabolites (CYP2D6; dextrorphan, CYP3A4; 1-hydroxymidazolam or 6β hydroxytestosterone) using HPLC-MS/MS and setting the value of the activity of an enzyme when drug was not incorporated therein as 100%. 50% inhibitory concentration IC50 (µM) was calculated by drawing a straight line between the concentration where the inhibition rate exceeds 50% and the concentration where the inhibition rate is less than 50 % in a graph showing a relationship between the inhibition rate and the concentration and interpolating the concentration that becomes 50% inhibition.

As a part of the test results, the inhibitory activity in the respective enzymes of the compound 1-1 of the present invention is shown in Table 6. In addition, the values in parentheses show the inhibition rates (%) in the shown concentration.

### [Table 10]

**Table 6**

| | IC50(µM) | | |
|---|---|---|---|
| Isozyme | CYP2D6 | CYP3A4 | |
| Substrate | Dextromethorphan | Midazolam | Testosterone |
| 1-1 | >10 (36%) | >10 (28%) | >10 (6%) |

The compound of the present invention did not show an inhibitory action of exceeding 50% in 10 µM with respect to CYP2D6 and CYP3A4 serving as a major human drug-metabolizing enzyme. This indicates that a drug interaction caused by taking the compound of the present invention and another agent is small and this becomes a clinical advantage.

As described above, the compound of the present invention is useful as an active ingredient of therapeutic medicine or preventive medicine for retinochoroidal disorders, particularly, therapeutic medicine or preventive medicine for retinochoroidal diseases accompanied by retinal light disorders. The therapeutic medicine or preventive medicine of the present invention maintains excellent tissue migration properties through oral administration and therapeutic effects or preventive effects can be expected with respect to the retinal light disorders.

### (15-lipoxygenase activity inhibitory action)

The inhibitory action of the compound of the present invention with respect to the activity of the present enzyme was examined using rabbit reticulocyte-derived 15-lipoxygenase.

Rabbit reticulocyte-derived 15-lipoxygenase was reacted with 260 µM of linoleic acid and the amount of 13-hydroperoxide which was a reaction product was measured using an absorption spectrometer. 3 µM of the compound of the present invention dissolved in dimethyl sulfoxide was added to the reaction system and the inhibition rate of 13-hydroperoxide generation (15-lipoxygenase activity inhibition rate) was measured.

From among several measurement results, the 15-lipoxygenase activity inhibition rate of the compound 1-1 at a concentration of 3 µM according to the present invention was 49%. That is, the 50% inhibitory concentration with respect to 15-lipoxygenase activity was approximately 3 µM.

Since 15-lipoxygenase is an enzyme that oxidizes the lipid, oxidation of a cell membrane is suppressed and the cell membrane is protected (Journal of Neuroscience Research, 2008; vol. 86; pp. 904 to 909). In addition, since 15-lipoxygenase is an enzyme related to generation of prostaglandin which is a mediator of an inflammatory reaction (Progress in Lipid Research, 2006; vol. 45; pp. 334 to 356), the progress of the inflammatory reaction can be reduced by inhibiting the present enzyme activity. As described above, it can be expected that cells are protected and the progress of inflammation is reduced through a 15-lipoxygenase activity inhibitory action by administering the compound of the present invention to a patient having an inflammatory disease.

### (Leukotriene A₄ hydrolase activity inhibitory action)

The inhibitory action of the compound according to the present invention with respect to the activity of the present enzyme was examined using guinea pig lung-derived leukotriene A₄ hydrolase.

Dunkin-Hartley guinea pig lung-derived leukotriene A₄ hydrolase was reacted with 1.70 µg/mL of leukotriene A₄ and the amount of leukotriene B₄ which was a reaction product was measured by an enzyme-linked immunosorbent assay. 3 µM of the compound of the present invention dissolved in dimethyl sulfoxide was added to the reaction system and the inhibition rate of leukotriene B₄ generation (leukotriene A₄ hydrolase activity inhibition rate) was measured.

From among several measurement results, the leukotriene A₄ hydrolase activity inhibition rate of the compound 1-1 according to the present invention at a concentration of 3 µM was 14%.

The leukotriene A₄ hydrolase is an enzyme that generates leukotriene B₄, and the leukotriene B₄ is a strong chemical inducer that activates neutrophils and monocytes and has an action of accumulating these cells. Thus, the activation of inflammatory cells is suppressed and the progress of inflammation can be suppressed (Agents Actions, 1986; vol. 17; pp. 366 to 367) by inhibiting the present enzyme activity. As described above, it can be expected that the progress of inflammation is suppressed through a leukotriene A₄ hydrolase activity inhibitory action by administering the compound of the present invention to a patient with an inflammatory disease.

### (Leukotriene C₄ synthase activity inhibitory action)

The inhibitory action according to the compound of the present invention with respect to the activity of the present enzyme was examined using guinea pig lung-derived leukotriene C₄ synthase.

Dunkin-Hartley guinea pig lung-derived leukotriene C₄ synthase was reacted with 2.50 µg/mL of leukotriene A₄ and the amount of leukotriene C₄ which was a reaction product was measured by an enzyme-linked immunosorbent assay. 3 µM of the compound of the present invention dissolved in dimethyl sulfoxide was added to the reaction system and the inhibition rate of leukotriene C₄ generation (leukotriene C₄ synthase activity inhibition rate) was measured.

From among several measurement results, the leukotriene C₄ synthase activity inhibition rate of the compound 1-1 according to the present invention at a concentration of 3 µM was 27%.

The leukotriene C₄ synthase is an enzyme that generates leukotriene C₄, and the leukotriene C₄ causes a contraction of the vascular smooth muscle. In addition, the leukotriene C₄ promotes a secretion of mucus to an airway or a visceral tissue, and aggregates white blood cells to an inflamed region by improving the vascular permeability of microvessels. Therefore, vascular contraction and the progress of inflammation can be suppressed by inhibiting the present enzyme activity (Biochemical Pharmacology, 1985; vol. 34; pp. 2695 to 2704). As described above, it can be expected that the progress of inflammation is suppressed through a leukotriene C₄ synthase activity inhibitory action by administering the compound of the present invention to a patient with an inflammatory disease and nutrient supplies to tissues and cells are secured by suppressing the vascular contraction.

### (Thromboxane synthase activity inhibitory action)

The inhibitory action of the compound according to the present invention with respect to the activity of the present enzyme was examined using human platelet-derived thromboxane synthase.

Thromboxane synthase of human recombinant cells was reacted with 2.10 µM of prostaglandin H₂ and the amount of thromboxane B₂ which was a reaction product was measured by an enzyme-linked immunosorbent assay. 3 µM of the compound according to the present invention dissolved in dimethyl sulfoxide was added to the reaction system and the inhibition rate of thromboxane B₂ generation (thromboxane synthase activity inhibition rate) was measured.

From among several measurement results, the thromboxane synthase activity inhibition rate of the compound 1-1 according to the present invention at a concentration of 3 µM was 32%.

The thromboxane synthase is an enzyme that generates thromboxane A₂, and the thromboxane A₂ causes a contraction of the vascular smooth muscle. In addition, the thromboxane A₂ improves secretion of mucus to an airway or a visceral tissue, and aggregates white blood cells to an inflamed region by improving the vascular permeability of microvessels. Therefore, vascular contraction and the progress of inflammation can be suppressed by inhibiting the present enzyme activity. As described above, it can be expected that the progress of inflammation is suppressed through a thromboxane synthase activity inhibitory action by administering the compound of the present invention to a patient with an inflammatory disease. and nutrient supply to cells is secured by suppressing the vascular contraction.

### (Phospholipase A₂-II activity inhibitory action)

The inhibitory action of the compound according to the present invention with respect to the activity of the present enzyme was examined using rattlesnake-derived phospholipase A₂-II.

The rattlesnake-derived phospholipase A₂-II was reacted with 0.030 µCi of 1-palmitoyl-2-[1-¹⁴C]oleoyl-L-3-phosphatidylcholine and the amount of ¹⁴C oleate which was a reaction product was measured by a radiation measurement method. 3 µM of the compound according to the present invention dissolved in dimethyl sulfoxide was added to the reaction system and the inhibition rate of ¹⁴C oleate generation (phospholipase A₂-II activity inhibition rate) was measured.

From among several measurement results, the phospholipase A₂-II activity inhibition rate of the compound 1-1 according to the present invention at a concentration of 3 µM was 12%.

Since the phospholipase A₂-II is an enzyme related to generation of prostaglandin which is a mediator of an inflammatory reaction, the progress of the inflammation reaction can be suppressed by inhibiting the present enzyme activity. As described above, it can be expected that cells are protected and the progress of inflammation is suppressed through a phospholipase A₂-II activity inhibitory action by administering the compound of the present invention to a patient with an inflammatory disease.

### (Lipid peroxidase activity inhibitory action)

The inhibitory action of the compound according to the present invention with respect to the activity of the present enzyme was examined using guinea pig liver microsome-derived lipid peroxidase.

Dunkin-Hartley guinea pig lung-derived lipid peroxidase was reacted with 0.25 M of polyvalent unsaturated fatty acids and the amount of malondialdehyde which was a reaction product was measured by an absorbance determination method. 3 µM of the compound according to the present invention dissolved in dimethyl sulfoxide was added to the reaction system and the inhibition rate of malondialdehyde generation (lipid peroxidase activity inhibition rate) was measured.

From among several measurement results, the lipid peroxidase activity inhibition rate of the compound 1-1 according to the present invention at a concentration of 3 µM was 13%.

Since the lipid peroxidase excessively oxidizes lipids, the progress of oxidation of cell membranes can be suppressed by inhibiting the present enzyme activity. As described above, it can be expected that cells are protected through a lipid peroxidase activity inhibitory action by administering the compound of the present invention to a patient with a disease caused by lipid oxidation.

### (MAP kinase 1 activity inhibitory action)

The inhibitory action of the compound of the present invention with respect to the activity of the present enzyme was examined using human recombinant MAP kinase 1.

The human recombinant MAP kinase 1 was reacted with 50.0 µg/mL of myelin basic protein and the amount of ³²P phosphorylated myelin basic protein which was a reaction product was measured by a radioactivity measurement method. 3 µM of the compound according to the present invention dissolved in dimethyl sulfoxide was added to the reaction system and the inhibition rate of ³²P phosphorylated myelin basic protein generation (MAP kinase 1 activity inhibition rate) was measured.

From among several measurement results, the MAP kinase 1 activity inhibition rate of the compound 1-1 according to the present invention at a concentration of 3 µM was 13%.

The MAP kinase 1 induces proliferation or differentiation of cells. Thus, when the compound of the present invention is administered to a patient with an inflammatory disease, it can be expected that the progress of inflammation is suppressed by inhibiting differentiation or proliferation to inflammatory lymphocytes in an inflamed region through the MAP kinase 1 activity inhibitory action.

As described above, since the compound of the present invention inhibits of the activities of various enzymes worsening the inflammatory reaction, the compound is useful as an active ingredient of therapeutic medicine or preventive medicine for various inflammatory diseases. Further, since the compound of the present invention inhibits of the activity of a lipid oxidation enzyme, the compound is useful as an active ingredient of therapeutic medicine or preventive medicine for various diseases caused by lipid oxidation. The therapeutic medicine or preventive medicine of the present invention maintains excellent tissue migration properties through oral administration and therapeutic effects or preventive effects can be expected with respect to inflammatory diseases and various diseases caused by lipid oxidation.

### Reference Signs List

- 1:: retinal pigment epithelial layer
- 2:: visual cell layer
- 3:: external limiting membrane
- 4:: ONL
- 5:: outer plexiform layer
- 6:: inner nuclear layer
- 7:: inner plexiform layer
- 8:: ganglion cell layer
- 9:: optic nerve fiber layer
- 10:: internal limiting membrane
- 11:: optic part of retina
- 12:: ciliary part of retina
- 13:: iridial part of retina
- 14:: retina
- 15:: choroid
- 16:: sclera
- 17:: optic nerve papilla
- 18:: macular region
- 19:: central fovea

## Claims

1. A phenylimidazole derivative represented by Formula (I) or (II), or a salt thereof, 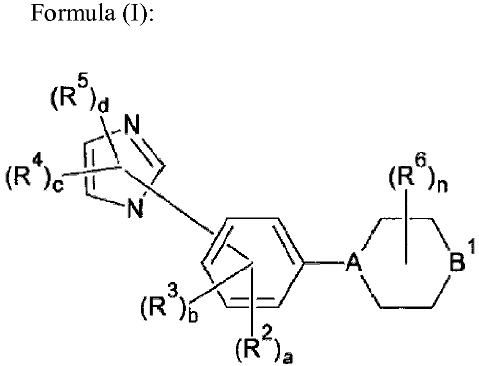 (in Formula (I), A represents a carbon atom or a nitrogen atom; B¹ represents a carbonyl group, a group represented by N-COR^{1a}, a group represented by N-C(R^{1a})=NOH, or a group represented by N-SO₂R ^{1b}; R^{1a} represents a hydrogen atom, an alkyl group having 1 to 6 carbon atoms, an alkyl group which is substituted with G¹ and has 1 to 6 carbon atoms, an alkenyl group having 2 to 6 carbon atoms, an alkenyl group which is substituted with G1 and has 2 to 6 carbon atoms, an alkynyl group having 2 to 6 carbon atoms, an alkynyl group which is substituted with G¹ and has 2 to 6 carbon atoms, a cycloalkyl group having 3 to 8 carbon atoms, a cycloalkyl group which is substituted with G¹ and has 3 to 8 carbon atoms, a hydroxyl group, an alkoxy group having 1 to 6 carbon atoms, an alkoxy group which is substituted with G¹ and has 1 to 6 carbon atoms, an alkenyloxy group having 2 to 6 carbon atoms, an alkenyloxy group which is substituted with G¹ and has 2 to 6 carbon atoms, an alkynyloxy group having 2 to 6 carbon atoms, an alkynyloxy group which is substituted with G¹ and has 2 to 6 carbon atoms, an amino group, an amino group substituted with one G², or an amino group substituted with two G²s which are the same as or different from each other (in a case where the amino group is substituted with two G²s, two G²s may be bonded to each other to form a ring); R^{1b} represents an alkyl group having 1 to 6 carbon atoms, an alkyl group which is substituted with G¹ and has 1 to 6 carbon atoms, an alkenyl group having 2 to 6 carbon atoms, an alkenyl group which is substituted with G² and has 2 to 6 carbon atoms, an alkynyl group having 2 to 6 carbon atoms, an alkynyl group which is substituted with G¹ and has 2 to 6 carbon atoms, a cycloalkyl group having 3 to 8 carbon atoms, a cycloalkyl group which is substituted with G¹ and has 3 to 8 carbon atoms, a hydroxyl group, an alkoxy group having 1 to 6 carbon atoms, an alkoxy group which is substituted with G¹ and has 1 to 6 carbon atoms, an alkenyloxy group having 2 to 6 carbon atoms, an alkenyloxy group which is substituted with G¹ and has 2 to 6 carbon atoms, an alkynyloxy group having 2 to 6 carbon atoms, an alkynyloxy group which is substituted with G¹ and has 2 to 6 carbon atoms, an amino group, an amino group substituted with one G², or an amino group substituted with two G²s which are the same as or different from each other (in a case where the amino group is substituted with two G²s, two G²s may be bonded to each other to form a ring); G¹ represents a halogen atom, a hydroxyl group, an alkoxy group having 1 to 6 carbon atoms, an amino group, or an amino group substituted with an alkyl group having 1 to 6 carbon atoms; G² represents an alkyl group having 1 to 6 carbon atoms, an alkyl group which is substituted with G¹ and has 1 to 6 carbon atoms, an alkenyl group having 2 to 6 carbon atoms, an alkenyl group which is substituted with G¹ and has 2 to 6 carbon atoms, an alkynyl group having 2 to 6 carbon atoms, an alkynyl group which is substituted with G¹ and has 2 to 6 carbon atoms, a cycloalkyl group having 3 to 8 carbon atoms, a cycloalkyl group which is substituted with G¹ and has 3 to 8 carbon atoms, an alkylidene group having 1 to 6 carbon atoms, an alkylidene group which is substituted with G¹ and has 1 to 6 carbon atoms, a formyl group, an alkylcarbonyl group having 1 to 6 carbon atoms, an alkylcarbonyl group which is substituted with G¹ and has 1 to 6 carbon atoms, a cycloalkylcarbonyl group having 3 to 8 carbon atoms, a cycloalkylcarbonyl group which is substituted with G¹ and has 3 to 8 carbon atoms, an alkoxycarbonyl group having 1 to 6 carbon atoms, an alkoxycarbonyl group which is substituted with G¹ and has 1 to 6 carbon atoms, an alkylsulfonyl group having 1 to 6 carbon atoms, or an alkylsulfonyl group which is substituted with G¹ and has 1 to 6 carbon atoms; R² represents an amino group, an amino group substituted with one G², an amino group substituted with two G²s which are the same as or different from each other, a hydroxyl group, an alkoxy group having 1 to 6 carbon atoms, an alkylcarbonyloxy group having 1 to 6 carbon atoms, an alkoxycarbonyloxy group having 1 to 6 carbon atoms, a cyano group, or an alkyl group which is substituted with G³ and has 1 to 6 carbon atoms; G³ represents an amino group, an amino group substituted with one G², an amino group substituted with two G²s which are the same as or different from each other, a hydroxyl group, an alkoxy group having 1 to 6 carbon atoms, an alkylcarbonyloxy group having 1 to 6 carbon atoms, an alkoxycarbonyloxy group having 1 to 6 carbon atoms, or a cyano group; a represents an integer of 1 to 4, and when a represents 2 or greater, R²s may be the same as or different from each other; R³ represents a halogen atom or an organic group other than G³; b represents an integer of 0 to 3, and when b represents 2 or greater, R³s may be the same as or different from each other, provided that a relationship of "a + b ≤ 4" is satisfied; R⁴ represents a cyano group or an alkyl group which is substituted with G³ and has 1 to 6 carbon atoms; c represents an integer of 0 to 3, and when c represents 2 or greater, R⁴s may be the same as or different from each other; R⁵ represents a halogen atom or an organic group other than G³; d represents an integer of 0 to 3, and when d represents 2 or greater, R⁵s may be the same as or different from each other, provided that a relationship of "c + d ≤ 3" is satisfied; R⁶ represents an alkyl group having 1 to 6 carbon atoms; and n represents an integer of 0 to 4, and when n represents 2 or greater, R⁶s may be the same as or different from each other and two R⁶s may be bonded to each other to form an alkylene group having 2 to 6 carbon atoms), and 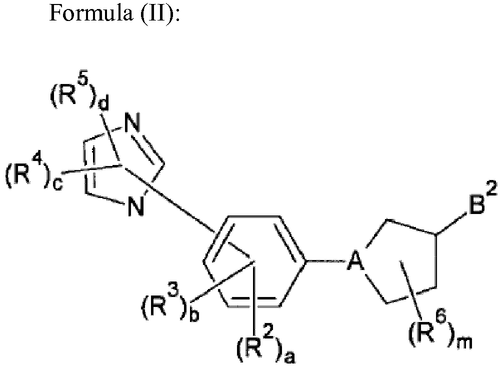 (in Formula (II), B² represents a group represented by NR^{1c}-COR^{1a}, a group represented by NR^{1c}-C(R^{1a})=NOH, or a group represented by NR^{1c}-SO₂R^{1b}; R^{1c} represents a hydrogen atom or an alkyl group having 1 to 6 carbon atoms; A, R^{1a}, R^{1b}, R², a, R³, b, R⁴, c, R⁵, d, and R⁶ have the same definitions as those in Formula (I); m represents an integer of 0 to 3, and when m represents 2 or greater, R⁶s may be the same as or different from each other and two R⁶s may be bonded to each other to form an alkylene group having 2 to 6 carbon atoms).

2. The phenylimidazole derivative according to Claim 1 or a salt thereof, wherein, in Formula (I), A represents a nitrogen atom; B¹ represents a group represented by N-COR^{1a}; and the imidazolyl group which is a substituent of the benzene ring is an imidazole-1-yl group.

3. The phenylimidazole derivative according to Claim 1 or a salt thereof,
wherein, in Formula (I), A represents a nitrogen atom; B¹ represents a group represented by N-COR^{1a}; the imidazolyl group which is a substituent of the benzene ring is an imidazole-1-yl group; and the imidazolyl group is in a meta-position with respect to a piperazine ring.

4. The phenylimidazole derivative according to Claim 1 or a salt thereof,
wherein, in Formula (II), A represents a nitrogen atom; B² represents a group represented by NR^{1c}-COR^{1a}; and the imidazolyl group which is a substituent of the benzene ring is an imidazole-1-yl group.

5. The phenylimidazole derivative according to Claim 1 or a salt thereof,
wherein an imidazolyl group, in Formula (II), A represents a nitrogen atom; B² represents a group represented by NR^{1c}-COR^{1a}; the imidazolyl group which is a substituent of the benzene ring is an imidazole-1-yl group; and the imidazolyl group is in a meta-position with respect to a pyrrolidine ring.

6. A therapeutic medicine or preventive medicine for inflammatory diseases, diseases caused by lipid oxidation, or retinochoroidal disorders, the medicine comprising at least one selected from the phenylimidazole derivative according to any one of Claims 1 to 5, a salt thereof, and metabolites thereof, as an active ingredient.

7. The therapeutic medicine or preventive medicine according to Claim 6, further comprising a pharmacologically acceptable additive.

8. The therapeutic medicine or preventive medicine according to Claim 7,
wherein the retinochoroidal disorder is age-related macular degeneration, diabetic retinopathy, or diabetic macular edema.

9. Use of at least one selected from the phenylimidazole derivative according to any one of Claims 1 to 5 and a salt thereof for treating or preventing inflammatory diseases, diseases caused by lipid oxidation, or retinochoroidal disorders.
